# EUROPEAN PATENT SPECIFICATION

(11) **EP 3 920 816 B1**
(45) Date of publication and mention of the grant of the patent: **20.03.2024**
(21) Application number: 20709998.7
(22) Date of filing: 11.02.2020
(51) Int. Cl.: A61B 17/70, A61B 17/80, A61B 17/86

(54) **PRESS-FIT ANTERIOR CERVICAL PLATE**
ANTERIORE ZERVIKALE PRESS-FIT-PLATTE
PLAQUE CERVICALE ANTÉRIEURE À AJUSTEMENT SERRÉ

(30) Priority: 11.02.2019 US 201962804049 P; 10.02.2020 US 202016786878
(43) Date of publication of application: 15.12.2021
(73) Proprietor: Nexus Spine, LLC, Salt Lake City, Utah 84121 (US)
(72) Inventor: MANWILL, Daniel, Salt Lake City, Utah 84121 (US); HALVERSON, Peter, Salt Lake City, Utah 84121 (US); HAWKES, David T., Salt Lake City, Utah 84121 (US)
(74) Representative: Dummett Copp LLP
(86) International application number: PCT/US2020/017692
(87) International publication number: WO 2020/167786

(56) References cited:
- US-A1- 2005 049 595
- US-A1- 2008 154 312
- US-A1- 2009 157 121
- US-A1- 2014 200 613
- US-A1- 2016 242 925

## Description

### TECHNICAL FIELD

The present invention relates to bone fixation devices, and more particularly to anterior cervical plates and systems.

### BACKGROUND ART

In the treatment of various spinal conditions, including the treatment of fractures, tumors, and degenerative conditions, it is necessary to secure and stabilize the anterior column of the spine following removal of a vertebral body or part. Various devices for internal fixation of bone segments in the human or animal body are known in the art.

Following such removal made using a thoracotomy, thoracoabdominal, retroperitoneal, or similar approach, the normal anatomy is reconstructed using tricortical iliac crest or fibular strut grafts. Not only are removals performed on the thoracic spine, as is the case for the above procedures, but also the cervical spine. Once bone matter is removed, it is then necessary to secure and stabilize the graft, desirably in such a manner as to permit rapid mobilization of the patient. Such objectives can be accomplished by a bone plate. However, to accomplish this service in the optimum manner, it is necessary that the plate be reasonably congruent with the bone to which it is applied, that it has as low a profile as possible, that it be firmly secured to the spinal column so that it is not torn out when the patient places weight and stress upon it and that it be capable of placement and fixation in a manner that is convenient for the surgeon.

In this context it is necessary to secure the plate to the spinal body and also, in some cases, to the graft. After the insertion of a graft and a plate, the graft placed in the patient tends to subside. Traditional cervical plates are designed to limit motion within the fusion mass. However, a German doctor by the surname of Wolff demonstrated that bone grows when in compression and resorbs in the absence thereof. Consequently, cervical plate technology has attempted to limit motion of the coupled spinal areas in all directions but compression; theorizing that the natural weight of the head would provide sufficient load to stimulate bone growth in the fusion mass. As disclosed in U.S. Patent No. 7,993,380 to Hawkes, certain cervical plates have been provided that provide active compression to supplement passive compression provided by the patient's head weight.

An orthopedic plate and screw system with the features as defined in the preamble of claim 1 is known from US 2016/242925 A1.

Unfortunately, previous orthopedic plates, including anterior cervical plates, have numerous disadvantages in their configuration and use. The prior systems require a separate system for distraction during the removal of the vertebral body or part. Prior plates are difficult to locate properly on the vertebrae due to limited visualization, and often require the use of specialized instruments and Caspar pins. The use of removable Caspar pins increases bone damage and bleeding that requires further treatment. It is also difficult to correctly size prior plates, and limitations on available sizes may require less than ideal plate placement.

With prior systems, cumbersome drill and screw guides are often needed to place and angle screws correctly relative to the plate. Furthermore, even once holes are drilled in the vertebrae, the introduction of the prior plates into the wound interferes with visualization needed to achieve proper screw placement. Even today, most orthopedic plates fail to adjust to bone remodeling or subsidence. Prior plates utilize separate screw-plate retention mechanisms. Such retention mechanisms add bulk, increase costs, add surgical steps, and may restrict screw angulation during placement.

For all these reasons, existing orthopedic plates, including cervical plates, include deficiencies that reduce positive outcomes for patients and cause difficulties for surgeons. It would be an improvement for orthopedic plates to address these deficiencies.

### BRIEF SUMMARY OF THE INVENTION

The invention is defined in claim 1 while preferred embodiments are set forth in the dependent claims.

Associated surgical methods are also described herein to aid understanding the invention.

These methods do not form part of the claimed invention. The present invention provides an orthopedic plate and screw system that addresses deficiencies of prior orthopedic plates, plate systems, and methods of use thereof. Orthopedic plates as described herein include anterior cervical plates. The new plates and plate systems allow screws to be placed with full visualization. The increased visualization allows screw placement even without use of specialized locating instruments or pins. The new plates are introduced to the surgical wound after the screws are placed; the wound is empty during screw placement when compared with prior screw placement methods. Additionally, because the screws are placed before the plates are introduced, the screws function as attachment points for distraction implements, and separate Caspar pins are not required.

The new plates and plate systems also obviate the need to achieve a particular position and angulation of screws. The screws allow more angulation than many prior devices allowed, and plate eyes of implementations of the invention adjust to screw position.

New plates in accordance with implementations of the invention include plate eyes that translate so as to match the effective plate size to the screw placement, thereby allowing each plate to fit multiple screw spacings. The new plates thereby reduce or eliminate difficulties associated with properly sizing prior art plates.

In accordance with implementations of the invention, plates are adapted to adjust to bone remodeling or subsidence. Screw eyes can slide to maintain graft contact. Some implementations of the invention provide unidirectional regulation of eye movement to help maintain graft compression.

Implementations of the invention reduce system bulk, as typical screw-plate retention mechanisms are not necessary. No ring, propeller, or wire screw retention mechanisms need be located above the screw heads. Implementations of the invention also decrease costs. New plates and plate systems have reduced locking-related parts count as screw retention is inherent rather than secondary. Implementations of the invention also reduce surgical steps. Caspar pin placement, pre-drilling of screw holes, actuating of screw locking mechanisms, and/or treatment of bone bleeding are eliminated using the orthopedic plate and screw system in accordance with implementations of the invention. Implementations of the invention permit a greater range of screw angulation during placement. Retention of the screws in implementations of the plate is inherent and independent of screw angulation up to the point of the screw neck contacting the plate; a wide range of screw angulation is achieved without the screw neck contacting the plate.

According to implementations of the invention, an orthopedic plate is provided. In some implementations, the orthopedic plate is a cervical plate for a cervical spine fixation procedure. The orthopedic plate includes an eye member. The eye member includes a biocompatible material formed to define a cylindrical passage through the eye member, the cylindrical passage being sized to receive an orthopedic screw head therein and to provide an interference fit with the orthopedic screw head. The eye member also includes a first frame member engagement contour and a second frame member engagement contour formed on opposite sides of the eye member. The orthopedic plate also includes a plate member. The plate member includes a channel formed by a first frame leg and a second frame leg, both of a biocompatible material. The first frame leg includes a first eye member engagement contour formed on a channel side of the first fame leg that is adapted to engage the first frame member engagement contour of the eye member in sliding engagement. The second frame leg includes a second eye member engagement contour formed on a channel side of the second fame leg that is adapted to engage the second frame member engagement contour of the eye member in sliding engagement. The eye member is contained within and adapted to slide within the channel.

In some implementations, the first frame member engagement contour and the second frame member engagement contour each include an extension extending laterally away from a center of the eye member, and the first eye member engagement contour and the second eye member engagement contour each include a slot sized and shaped to slidingly receive one extension of the eye member. In some implementations, the extension of the first frame member engagement contour and the second frame member engagement contour includes a cross-sectional shape such as a rectangular shape, a rectangular shape with rounded corners, a dovetail shape, a partially circular shape, a partially ellipse shape, a triangular shape, a trapezoidal shape, a polygonal shape, or a shape with one or more out-of-plane protrusions.

In some implementations, the first eye member engagement contour and the second eye member engagement contour each include an extension extending laterally toward the other frame leg, and the first frame member engagement contour and the second frame member engagement contour each include a slot sized and shaped to slidingly receive one extension of the plate member. In some implementations the extension of the first eye member engagement contour and the second eye member engagement contour includes a cross-sectional shape such as a rectangular shape, a rectangular shape with rounded corners, a dovetail shape, a partially circular shape, a partially ellipse shape, a triangular shape, a trapezoidal shape, a polygonal shape, or a shape with one or more out-of-plane protrusions. The orthopedic plate of claim 1, wherein the plate member further includes a static cylindrical passage sized to receive another orthopedic screw head therein and to provide an interference fit with the other orthopedic screw head.

Some implementations further include another eye member. The other eye member includes a biocompatible material formed to define a cylindrical passage through the other eye member, the cylindrical passage through the other eye member being sized to receive another orthopedic screw head therein and to provide an interference fit with the other orthopedic screw head. The other eye member also includes a third frame member engagement contour and a fourth frame member engagement contour formed on opposite sides of the other eye member. In such embodiments, the plate member further includes another channel formed by a third frame leg and a fourth frame leg, both of a biocompatible material. The third frame leg includes a third eye member engagement contour formed on a channel side of the third fame leg that is adapted to engage the third frame member engagement contour of the other eye member in sliding engagement. The fourth frame leg includes a fourth eye member engagement contour formed on a channel side of the fourth fame leg that is adapted to engage the fourth frame member engagement contour of the other eye member in sliding engagement. The other eye member is contained within and adapted to slide within the other channel.

Some implementations also include another eye member. The other eye member includes a biocompatible material formed to define a cylindrical passage through the other eye member, the cylindrical passage through the other eye member being sized to receive another orthopedic screw head therein and to provide an interference fit with the other orthopedic screw head. The other eye member also includes a third frame member engagement contour and a fourth frame member engagement contour formed on opposite sides of the other eye member that are adapted to engage the first and second eye member engagement contours of the first and second frame legs. The other eye member is contained within and adapted to slide within the channel (the channel formed by the first and second legs).

According to some implementations, the orthopedic plate also includes a force-generating mechanism adapted to apply a compressive force between the eye member and the other eye member. According to some implementations, the force-generating mechanism includes one of a spring, a compliant mechanism, or a nickel-titanium alloy (nitinol) wire.

Some implementations also include orthopedic screws each having the orthopedic screw head sized to provide an interference fit with the cylindrical passage to thereby form an orthopedic plate system. In some implementations, each of the orthopedic screws includes a driving feature and a wand-attachment feature adapted to permit retention of the orthopedic screw against a force directed against the orthopedic screw head during interference fitting of the orthopedic screw head in the cylindrical passage.

A method of using the orthopedic plate systen (not claimed) includes steps of inserting one orthopedic screw into a first anterior portion of a vertebral body of a first vertebra and inserting another orthopedic screw into a second anterior portion of a vertebral body of a second vertebra. The method also includes using the orthopedic screws to distract the disc space for a discectomy and insertion of an interbody implant or graft. The method also includes determining a proper length for the plate member using a distance between the orthopedic screw heads and inserting and positioning the plate member in the wound with the cylindrical passage over one orthopedic screw head. The method further includes locking the plate member to one of the orthopedic screws by drawing the orthopedic screw head into the cylindrical passage of the eye member while pushing the eye member toward the orthopedic screw head until an interference fit is achieved between the orthopedic screw head and the eye member.

In some implementations, the orthopedic plate further including a motion-stopping structure adapted to at least selectively inhibit sliding motion of the eye member within the channel. In some implementations, the motion-stopping structure is a structure such as a cylindrical roller clutch structure, a sprag clutch structure, a ball clutch structure, a cylindrical roller clutch structure with an energizing spring, a sprag clutch structure with an energizing spring, a ball clutch structure with an energizing spring, a toothed ratchet mechanism, a self-energizing wedge, an integrated camming member, or an interference fit between the eye member and the channel caused by expansion of the eye member upon insertion of the orthopedic screw head into the cylindrical passage.

In some implementations, the channel has either an open-ended shape or a closed-ended shape. In some implementations, the orthopedic plate is sized for use in a cervical vertebra fixation procedure. In some implementations, the orthopedic plate is part of an orthopedic plate system including an orthopedic screw having the orthopedic screw head. In some implementations, the orthopedic plate system also includes one or more tools or instruments for driving the orthopedic screw and/or for applying force between the orthopedic screw and the plate member.

According to further implementation, an orthopedic plate system is provided. The orthopedic plate system is a cervical plate system for a cervical spine fixation procedure. The orthopedic plate system includes a first orthopedic screw having a first threaded shaft extending from a first orthopedic screw head and a second orthopedic screw having a second threaded shaft extending from a second orthopedic screw head. The orthopedic plate system also includes a first eye member. The first eye member includes a biocompatible material formed to define a first cylindrical passage through the first eye member, the first cylindrical passage being sized to receive the first orthopedic screw head therein and to provide an interference fit with the first orthopedic screw head. The first eye member also includes a first frame member engagement contour and a second frame member engagement contour formed on opposite sides of the first eye member.

The orthopedic plate system also includes a second eye member. The second eye member includes a biocompatible material formed to define a second cylindrical passage through the second eye member, the second cylindrical passage through the second eye member being sized to receive the second orthopedic screw head therein and to provide an interference fit with the second orthopedic screw head. The second eye member also includes a third frame member engagement contour and a fourth frame member engagement contour formed on opposite sides of the second eye member.

The orthopedic plate system also includes a plate member. The plate member includes a first channel formed by a first frame leg and a second frame leg, both of a biocompatible material. The first frame leg includes a first eye member engagement contour formed on a channel side of the first fame leg that adapted to engage the first frame member engagement contour of the first eye member in sliding engagement. The second frame leg includes a second eye member engagement contour formed on a channel side of the second fame leg that is adapted to engage the second frame member engagement contour of the first eye member in sliding engagement. The plate member also includes a second channel formed by a third frame leg and a fourth frame leg, both of a biocompatible material. The third frame leg includes a third eye member engagement contour formed on a channel side of the third fame leg that is adapted to engage the third frame member engagement contour of the second eye member in sliding engagement. The fourth frame leg includes a fourth eye member engagement contour formed on a channel side of the forth fame leg that is adapted to engage the fourth frame member engagement contour of the second eye member in sliding engagement. The first eye member is contained within and adapted to slide within the first channel and the second eye member is contained within and adapted to slide within the second channel.

According to some implementations, the orthopedic plate system further includes motion-stopping structures adapted to at least selectively inhibit sliding motion of the first and second eye members within the first and second channels. According to some implementations, the first frame leg, the second frame leg, the third frame leg, and the fourth frame leg are integrally formed from a unitary piece of biocompatible material. According to some implementations, the first channel and the second channel are unitarily formed as a continuous closed-loop channel that is divided by an insert affixed near a center of the continuous closed-loop channel.

According to some implementations, the orthopedic plate system also includes a force-generating mechanism adapted to apply a compressive force between the first eye member and the second eye member. According to some implementations, the force-generating mechanism includes one of a spring, a compliant mechanism, or a nickel-titanium alloy (nitinol) wire.

In some implementations, the first frame member engagement contour, the second frame member engagement contour, the third frame member engagement contour, and the fourth frame member engagement contour each includes an extension extending laterally away from a center of the respective eye member, and the first eye member engagement contour, the second eye member engagement contour, the third eye member engagement contour, and the fourth eye member engagement contour each include a slot sized and shaped to slidingly receive one extension of the respective eye member. In some implementations, the extension of the first frame member engagement contour, the second frame member engagement contour, the third frame member engagement contour, and the fourth frame member engagement contour includes a cross-sectional shape such as a rectangular shape, a rectangular shape with rounded corners, a dovetail shape, a partially circular shape, a partially ellipse shape, a triangular shape, a trapezoidal shape, a polygonal shape, or a shape with one or more out-of-plane protrusions.

In some implementations, the first eye member engagement contour, the second eye member engagement contour, the third eye member engagement contour, and the fourth eye member engagement contour each include an extension extending laterally toward the other frame leg, and the first frame member engagement contour, the second frame member engagement contour, the third frame member engagement contour, and the fourth frame member engagement contour each include a slot sized and shaped to slidingly receive one extension of the plate member. In some implementations the extension of the first eye member engagement contour, the second eye member engagement contour, the third eye member engagement contour, and the fourth eye member engagement contour includes a cross-sectional shape such as a rectangular shape, a rectangular shape with rounded corners, a dovetail shape, a partially circular shape, a partially ellipse shape, a triangular shape, a trapezoidal shape, a polygonal shape, or a shape with one or more out-of-plane protrusions. The orthopedic plate of claim 1, wherein the plate member further includes a static cylindrical passage sized to receive another orthopedic screw head therein and to provide an interference fit with the other orthopedic screw head.

In some implementations, each of the orthopedic screws includes a driving feature and a wand-attachment feature adapted to permit retention of the orthopedic screw against a force directed against the orthopedic screw head during interference fitting of the respective orthopedic screw head in the respective cylindrical passage.

In some implementations, the orthopedic plate further including a motion-stopping structure adapted to at least selectively inhibit sliding motion of each of the first eye member and the second eye member within their respective channels. In some implementations, the motion-stopping structure is a structure such as a cylindrical roller clutch structure, a sprag clutch structure, a ball clutch structure, a cylindrical roller clutch structure with an energizing spring, a sprag clutch structure with an energizing spring, a ball clutch structure with an energizing spring, a toothed ratchet mechanism, a self-energizing wedge, an integrated camming member, or an interference fit between the eye member and the channel caused by expansion of the respective eye member upon insertion of the respective orthopedic screw head into the respective cylindrical passage.

A method of using the orthopedic plate systen (not claimed) includes steps of inserting the first orthopedic screw into a first anterior portion of a vertebral body of a first vertebra and inserting the second orthopedic screw into a second anterior portion of a vertebral body of a second vertebra. The method also includes using the first orthopedic screw and the second orthopedic screw to distract the disc space for a discectomy and insertion of an interbody implant or graft. The method also includes determining a proper length for the plate member using a distance between the first orthopedic screw head and the second orthopedic screw head and inserting and positioning the plate member in the wound with the first cylindrical passage over the first orthopedic screw head and the second cylindrical passage over the second orthopedic screw head. The method further includes locking the plate member to the first orthopedic screw and the second orthopedic screw by drawing the first orthopedic screw head into the first cylindrical passage of the first eye member while pushing the first eye member toward the first orthopedic screw head until an interference fit is achieved between the first orthopedic screw head and the first eye member and drawing the second orthopedic screw head into the second cylindrical passage of the second eye member while pushing the second eye member toward the second orthopedic screw head until an interference fit is achieved between the second orthopedic screw head and the second eye member.

### BRIEF DESCRIPTION OF THE DRAWINGS

The features of the present invention will become more fully apparent from the following description and appended claims, taken in conjunction with the accompanying drawings. Understanding that these drawings depict only typical embodiments of the invention and are, therefore, not to be considered limiting of its scope, the invention will be described and explained with additional specificity and detail through the use of the accompanying drawings in which:
Figure 1 shows a perspective view of an embodiment of an orthopedic implant;
Figure 2 shows a perspective view of an embodiment of an orthopedic implant;
Figure 3 shows cross-sectional views of multiple embodiments of orthopedic implants;
Figure 4 shows a top, partially-transparent view of an embodiment of an orthopedic implant;
Figure 5 shows a top, partially-transparent view of an embodiment of an orthopedic implant;
Figure 6 shows a top view of an embodiment of an eye member of an embodiment of an orthopedic implant;
Figure 7 shows a perspective view of an embodiment of an orthopedic implant;
Figure 8 shows a perspective bottom view of an embodiment of an orthopedic implant;
Figure 9 shows a perspective view of an embodiment of an orthopedic implant;
Figure 10 shows a perspective view of an embodiment of an orthopedic screw for use with embodiments of orthopedic implants;
Figure 11 shows a side perspective view of placement of two orthopedic screws anteriorly in the cervical spine as part of a surgical procedure;
Figure 12 shows a front perspective view of introduction of an embodiment of an orthopedic implant to a surgical wound after placement of orthopedic screws as part of a surgical procedure;
Figure 13 shows a perspective view of an embodiment of an orthopedic implant illustrating a locking step to lock surgical screws to the orthopedic implant as part of a surgical procedure;
Figure 14 shows a perspective view of an embodiment of an orthopedic implant after locking of the implant to surgical screws *in situ* as part of a surgical procedure; and
Figure 15 shows use of a radiograph as part of a surgical procedure to determine size of an embodiment of an orthopedic implant.

### DETAILED DESCRIPTION OF THE INVENTION

A description of embodiments of the present invention will now be given with reference to the Figures. It is expected that the present invention may take many other forms and shapes, hence the following disclosure is intended to be illustrative and not limiting, and the scope of the invention should be determined by reference to the appended claims.

Embodiments of the invention provide orthopedic plate and screw systems that address deficiencies of prior orthopedic plates, plate systems, and methods of use thereof. Orthopedic plates as described herein include anterior cervical plates. The new plates and plate systems allow screws to be placed with full visualization. The increased visualization allows screw placement even without use of specialized locating instruments or pins. The new plates are introduced to the surgical wound after the screws are placed; the wound is empty during screw placement when compared with prior screw placement methods. Additionally, because the screws are placed before the plates are introduced, the screws function as attachment points for distraction implements, and separate Caspar pins are not required.

The new plates and plate systems also obviate the need to achieve a particular position and angulation of screws. The screws allow more angulation than many prior devices allowed, and plate eyes of embodiments of the invention adjust to screw position.

New plates in accordance with embodiments of the invention include plate eyes that translate so as to match the effective plate size to the screw placement, thereby allowing each plate to fit multiple screw spacings. The new plates thereby reduce or eliminate difficulties associated with properly sizing prior art plates.

In accordance with embodiments of the invention, plates are adapted to adjust to bone remodeling or subsidence. Screw eyes can slide to maintain graft contact. Some embodiments of the invention provide unidirectional regulation of eye movement to help maintain graft compression.

Embodiments of the invention reduce system bulk, as typical screw-plate retention mechanisms are not necessary. No ring, propeller, or wire screw retention mechanisms need be located above the screw heads. Embodiments of the invention also decrease costs. New plates and plate systems have reduced locking-related parts count as screw retention is inherent rather than secondary. Embodiments of the invention also reduce surgical steps. Caspar pin placement, pre-drilling of screw holes, actuating of screw locking mechanisms, and/or treatment of bone bleeding are eliminated using the orthopedic plate and screw system in accordance with embodiments of the invention. Embodiments of the invention permit a greater range of screw angulation during placement. Retention of the screws in embodiments of the plate is inherent and independent of screw angulation up to the point of the screw neck contacting the plate; a wide range of screw angulation is achieved without the screw neck contacting the plate.

According to embodiments of the invention, an orthopedic plate is provided. In some embodiments, the orthopedic plate is a cervical plate for a cervical spine fixation procedure. The orthopedic plate includes an eye member. The eye member includes a biocompatible material formed to define a cylindrical passage through the eye member, the cylindrical passage being sized to receive an orthopedic screw head therein and to provide an interference fit with the orthopedic screw head. The eye member also includes a first frame member engagement contour and a second frame member engagement contour formed on opposite sides of the eye member. The orthopedic plate also includes a plate member. The plate member includes a channel formed by a first frame leg and a second frame leg, both of a biocompatible material. The first frame leg includes a first eye member engagement contour formed on a channel side of the first fame leg that is adapted to engage the first frame member engagement contour of the eye member in sliding engagement. The second frame leg includes a second eye member engagement contour formed on a channel side of the second fame leg that is adapted to engage the second frame member engagement contour of the eye member in sliding engagement. The eye member is contained within and adapted to slide within the channel.

In some embodiments, the first frame member engagement contour and the second frame member engagement contour each include an extension extending laterally away from a center of the eye member, and the first eye member engagement contour and the second eye member engagement contour each include a slot sized and shaped to slidingly receive one extension of the eye member. In some embodiments, the extension of the first frame member engagement contour and the second frame member engagement contour includes a cross-sectional shape such as a rectangular shape, a rectangular shape with rounded corners, a dovetail shape, a partially circular shape, a partially ellipse shape, a triangular shape, a trapezoidal shape, a polygonal shape, or a shape with one or more out-of-plane protrusions.

In some embodiments, the first eye member engagement contour and the second eye member engagement contour each include an extension extending laterally toward the other frame leg, and the first frame member engagement contour and the second frame member engagement contour each include a slot sized and shaped to slidingly receive one extension of the plate member. In some embodiments the extension of the first eye member engagement contour and the second eye member engagement contour includes a cross-sectional shape such as a rectangular shape, a rectangular shape with rounded corners, a dovetail shape, a partially circular shape, a partially ellipse shape, a triangular shape, a trapezoidal shape, a polygonal shape, or a shape with one or more out-of-plane protrusions. The orthopedic plate of claim 1, wherein the plate member further includes a static cylindrical passage sized to receive another orthopedic screw head therein and to provide an interference fit with the other orthopedic screw head.

Some embodiments further include another eye member. The other eye member includes a biocompatible material formed to define a cylindrical passage through the other eye member, the cylindrical passage through the other eye member being sized to receive another orthopedic screw head therein and to provide an interference fit with the other orthopedic screw head. The other eye member also includes a third frame member engagement contour and a fourth frame member engagement contour formed on opposite sides of the other eye member. In such embodiments, the plate member further includes another channel formed by a third frame leg and a fourth frame leg, both of a biocompatible material. The third frame leg includes a third eye member engagement contour formed on a channel side of the third fame leg that is adapted to engage the third frame member engagement contour of the other eye member in sliding engagement. The fourth frame leg includes a fourth eye member engagement contour formed on a channel side of the fourth fame leg that is adapted to engage the fourth frame member engagement contour of the other eye member in sliding engagement. The other eye member is contained within and adapted to slide within the other channel.

Some embodiments also include another eye member. The other eye member includes a biocompatible material formed to define a cylindrical passage through the other eye member, the cylindrical passage through the other eye member being sized to receive another orthopedic screw head therein and to provide an interference fit with the other orthopedic screw head. The other eye member also includes a third frame member engagement contour and a fourth frame member engagement contour formed on opposite sides of the other eye member that are adapted to engage the first and second eye member engagement contours of the first and second frame legs. The other eye member is contained within and adapted to slide within the channel (the channel formed by the first and second legs).

According to some embodiments, the orthopedic plate also includes a force-generating mechanism adapted to apply a compressive force between the eye member and the other eye member. According to some embodiments, the force-generating mechanism includes one of a spring, a compliant mechanism, or a nickel-titanium alloy (nitinol) wire.

Some embodiments also include orthopedic screws each having the orthopedic screw head sized to provide an interference fit with the cylindrical passage to thereby form an orthopedic plate system. In some embodiments, each of the orthopedic screws includes a driving feature and a wand-attachment feature adapted to permit retention of the orthopedic screw against a force directed against the orthopedic screw head during interference fitting of the orthopedic screw head in the cylindrical passage.

A method of using the orthopedic plate system (not claimed) includes steps of inserting one orthopedic screw into a first anterior portion of a vertebral body of a first vertebra and inserting another orthopedic screw into a second anterior portion of a vertebral body of a second vertebra. The method also includes using the orthopedic screws to distract the disc space for a discectomy and insertion of an interbody implant or graft. The method also includes determining a proper length for the plate member using a distance between the orthopedic screw heads and inserting and positioning the plate member in the wound with the cylindrical passage over one orthopedic screw head. The method further includes locking the plate member to one of the orthopedic screws by drawing the orthopedic screw head into the cylindrical passage of the eye member while pushing the eye member toward the orthopedic screw head until an interference fit is achieved between the orthopedic screw head and the eye member.

In some embodiments, the orthopedic plate further including a motion- stopping structure adapted to at least selectively inhibit sliding motion of the eye member within the channel. In some embodiments, the motion-stopping structure is a structure such as a cylindrical roller clutch structure, a sprag clutch structure, a ball clutch structure, a cylindrical roller clutch structure with an energizing spring, a sprag clutch structure with an energizing spring, a ball clutch structure with an energizing spring, a toothed ratchet mechanism, a self-energizing wedge, an integrated camming member, or an interference fit between the eye member and the channel caused by expansion of the eye member upon insertion of the orthopedic screw head into the cylindrical passage.

In some embodiments, the channel has either an open-ended shape or a closed-ended shape. In some embodiments, the orthopedic plate is sized for use in a cervical vertebra fixation procedure. In some embodiments, the orthopedic plate is part of an orthopedic plate system including an orthopedic screw having the orthopedic screw head. In some embodiments, the orthopedic plate system also includes one or more tools or instruments for driving the orthopedic screw and/or for applying force between the orthopedic screw and the plate member.

According to a further embodiment, an orthopedic plate system is provided. The orthopedic plate system is a cervical plate system for a cervical spine fixation procedure. The orthopedic plate system includes a first orthopedic screw having a first threaded shaft extending from a first orthopedic screw head and a second orthopedic screw having a second threaded shaft extending from a second orthopedic screw head. The orthopedic plate system also includes a first eye member. The first eye member includes a biocompatible material formed to define a first cylindrical passage through the first eye member, the first cylindrical passage being sized to receive the first orthopedic screw head therein and to provide an interference fit with the first orthopedic screw head. The first eye member also includes a first frame member engagement contour and a second frame member engagement contour formed on opposite sides of the first eye member.

The orthopedic plate system also includes a second eye member. The second eye member includes a biocompatible material formed to define a second cylindrical passage through the second eye member, the second cylindrical passage through the second eye member being sized to receive the second orthopedic screw head therein and to provide an interference fit with the second orthopedic screw head. The second eye member also includes a third frame member engagement contour and a fourth frame member engagement contour formed on opposite sides of the second eye member.

The orthopedic plate system also includes a plate member. The plate member includes a first channel formed by a first frame leg and a second frame leg, both of a biocompatible material. The first frame leg includes a first eye member engagement contour formed on a channel side of the first fame leg that adapted to engage the first frame member engagement contour of the first eye member in sliding engagement. The second frame leg includes a second eye member engagement contour formed on a channel side of the second fame leg that is adapted to engage the second frame member engagement contour of the first eye member in sliding engagement. The plate member also includes a second channel formed by a third frame leg and a fourth frame leg, both of a biocompatible material. The third frame leg includes a third eye member engagement contour formed on a channel side of the third fame leg that is adapted to engage the third frame member engagement contour of the second eye member in sliding engagement. The fourth frame leg includes a fourth eye member engagement contour formed on a channel side of the forth fame leg that is adapted to engage the fourth frame member engagement contour of the second eye member in sliding engagement. The first eye member is contained within and adapted to slide within the first channel and the second eye member is contained within and adapted to slide within the second channel.

According to some embodiments, the orthopedic plate system further includes motion-stopping structures adapted to at least selectively inhibit sliding motion of the first and second eye members within the first and second channels. According to some embodiments, the first frame leg, the second frame leg, the third frame leg, and the fourth frame leg are integrally formed from a unitary piece of biocompatible material. According to some embodiments, the first channel and the second channel are unitarily formed as a continuous closed-loop channel that is divided by an insert affixed near a center of the continuous closed-loop channel.

According to some embodiments, the orthopedic plate system also includes a force-generating mechanism adapted to apply a compressive force between the first eye member and the second eye member. According to some embodiments, the force-generating mechanism includes one of a spring, a compliant mechanism, or a nickel-titanium alloy (nitinol) wire.

In some embodiments, the first frame member engagement contour, the second frame member engagement contour, the third frame member engagement contour, and the fourth frame member engagement contour each includes an extension extending laterally away from a center of the respective eye member, and the first eye member engagement contour, the second eye member engagement contour, the third eye member engagement contour, and the fourth eye member engagement contour each include a slot sized and shaped to slidingly receive one extension of the respective eye member. In some embodiments, the extension of the first frame member engagement contour, the second frame member engagement contour, the third frame member engagement contour, and the fourth frame member engagement contour includes a cross-sectional shape such as a rectangular shape, a rectangular shape with rounded corners, a dovetail shape, a partially circular shape, a partially ellipse shape, a triangular shape, a trapezoidal shape, a polygonal shape, or a shape with one or more out-of-plane protrusions.

In some embodiments, the first eye member engagement contour, the second eye member engagement contour, the third eye member engagement contour, and the fourth eye member engagement contour each include an extension extending laterally toward the other frame leg, and the first frame member engagement contour, the second frame member engagement contour, the third frame member engagement contour, and the fourth frame member engagement contour each include a slot sized and shaped to slidingly receive one extension of the plate member. In some embodiments the extension of the first eye member engagement contour, the second eye member engagement contour, the third eye member engagement contour, and the fourth eye member engagement contour includes a cross-sectional shape such as a rectangular shape, a rectangular shape with rounded corners, a dovetail shape, a partially circular shape, a partially ellipse shape, a triangular shape, a trapezoidal shape, a polygonal shape, or a shape with one or more out-of-plane protrusions. The orthopedic plate of claim 1, wherein the plate member further includes a static cylindrical passage sized to receive another orthopedic screw head therein and to provide an interference fit with the other orthopedic screw head.

In some embodiments, each of the orthopedic screws includes a driving feature and a wand-attachment feature adapted to permit retention of the orthopedic screw against a force directed against the orthopedic screw head during interference fitting of the respective orthopedic screw head in the respective cylindrical passage.

In some embodiments, the orthopedic plate further including a motion-stopping structure adapted to at least selectively inhibit sliding motion of each of the first eye member and the second eye member within their respective channels. In some embodiments, the motion-stopping structure is a structure such as a cylindrical roller clutch structure, a sprag clutch structure, a ball clutch structure, a cylindrical roller clutch structure with an energizing spring, a sprag clutch structure with an energizing spring, a ball clutch structure with an energizing spring, a toothed ratchet mechanism, a self-energizing wedge, an integrated camming member, or an interference fit between the eye member and the channel caused by expansion of the respective eye member upon insertion of the respective orthopedic screw head into the respective cylindrical passage.

A method of using the orthopedic plate system (not claimed) includes steps of inserting the first orthopedic screw into a first anterior portion of a vertebral body of a first vertebra and inserting the second orthopedic screw into a second anterior portion of a vertebral body of a second vertebra. The method also includes using the first orthopedic screw and the second orthopedic screw to distract the disc space for a discectomy and insertion of an interbody implant or graft. The method also includes determining a proper length for the plate member using a distance between the first orthopedic screw head and the second orthopedic screw head and inserting and positioning the plate member in the wound with the first cylindrical passage over the first orthopedic screw head and the second cylindrical passage over the second orthopedic screw head. The method further includes locking the plate member to the first orthopedic screw and the second orthopedic screw by drawing the first orthopedic screw head into the first cylindrical passage of the first eye member while pushing the first eye member toward the first orthopedic screw head until an interference fit is achieved between the first orthopedic screw head and the first eye member and drawing the second orthopedic screw head into the second cylindrical passage of the second eye member while pushing the second eye member toward the second orthopedic screw head until an interference fit is achieved between the second orthopedic screw head and the second eye member.

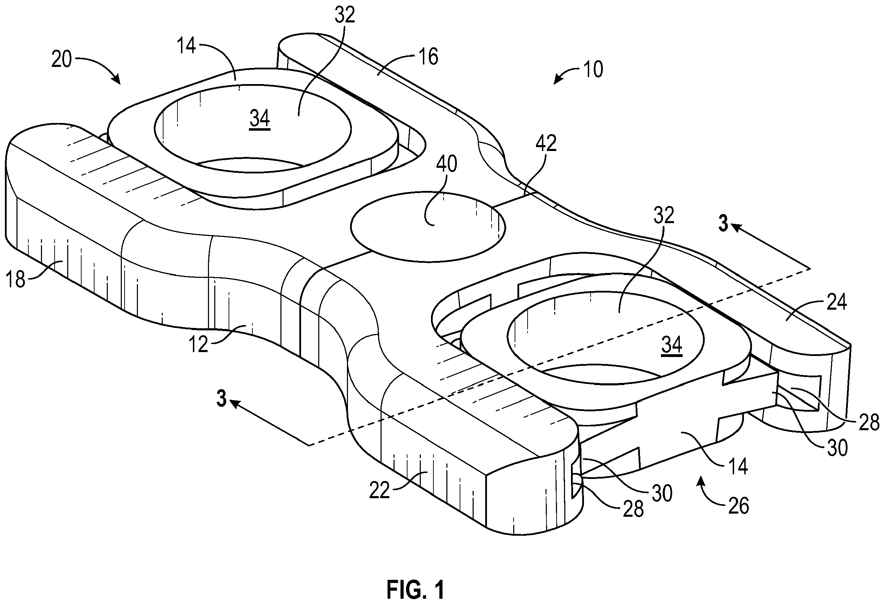

Figure 1 shows one embodiment of an orthopedic plate, which is illustrated as an anterior cervical plate 10. In this embodiment, the plate 10 includes a frame member 12 and two eye members 14. The frame member 12 and the eye member 14 or eye members 14 are all formed of biocompatible materials including, but in no way limited to, stainless steel, titanium, a titanium alloy such as, for example Ti 6-4 (approximately 6% aluminum, 4% vanadium, up to 0.25% iron, up to 0.2% oxygen and the remainder titanium), Ti 6-7 (approximately 6% aluminum and approximately 7% niobium), tantalum, a tantalum alloy, and other recognized alloys used for implants. In some embodiments, the frame member 12 and the eye member 14 or eye members 14 have similar compositions, and in other embodiments, the compositions of the frame member 12 and the eye member 14 or eye members 14 vary.

The frame member 12 of this embodiment of the plate 10 is formed as a unitary construction having a first leg 16 and a second leg 18 defining a first channel 20, and a third leg 22 and a fourth leg 24 defining a second channel 26. The first channel 20 receives and slidingly secures a first of the eye members 14 therein, and the second channel receives and slidingly secures a second of the eye members 14 therein. In some embodiments, the frame member 12 is substantially planar, and in other embodiments, the frame member 12 has a curve to it that generally matches a curve of an anticipated implant location, such as the anterior portion of the cervical spine.

To allow the first channel 20 and the second channel 26 to receive and slidingly secure the eye members 14 therein, each of the legs 16, 18, 22, and 24 has a channel or eye member engagement contour 28 formed on a channel side thereof (a channel-facing or channel defining side of the respective leg 16, 18, 22, or 24). The eye members 14 each have a corresponding frame member engagement contour 30 formed on opposite sides of the eye member 14 such that the contours 30 slidingly engage the contours 28 as shown in Figure 1. The sliding engagement of the contours 30 with the contours 28 allows the eye members 14 to move within the frame member 12 to adjust a spacing between the eye members 14.

This movement between eye members 14 provides several functions to the plate 10. First, the plate 10 shown in Figure 1 can be used to fit a range of spacings between orthopedic screws (not shown in Figure 1). A surgeon or hospital using the plate 10 therefore need not have as many available sizes of plates 10, as the movement between eye members 14 allows for a single plate 10 to encompass a range of spacing between orthopedic screws. Accordingly, the inventory requirements for the surgeon or hospital is reduced. Second, the measurement of the spacing between orthopedic screws need not be as precise as prior art orthopedic plates, as the movement between eye members 14 can compensate for a measure of imprecision. Third, the plate 10 permits the eye members 14 to move toward each other postoperatively to account for bone remodeling or subsidence post implant, thereby maintain graft compression (either passive due to weight of the head or active compression provided by a feature incorporated with plate 10). In some embodiments, the plate 10 is provided with an active compression mechanism or other force-generating mechanism that provides a gentle compressive force between the eye members 14 after implantation such as one or more springs, a compliant mechanism, a nitinol wire extending between or around the eye members 14, or the like. Embodiments of the plate 10 embrace any mechanism for applying a compressive force between the eye members 14.

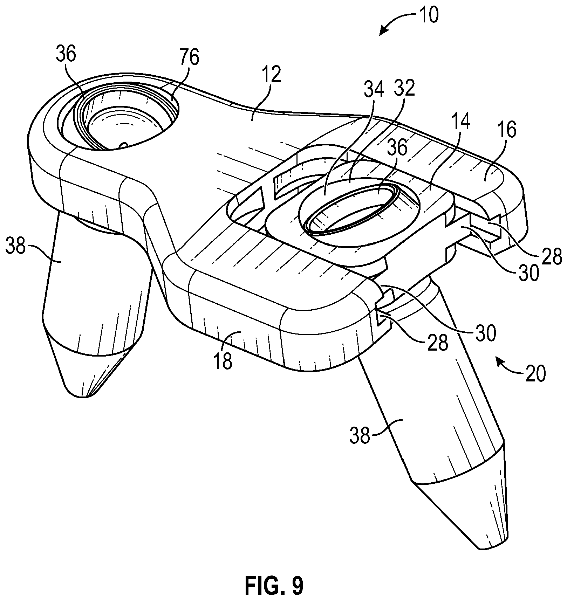

The eye members 14 each are formed as a unitary construction defining a cylindrical passage 32 therethrough. The cylindrical passage 32 is defined by an inner surface 34 of the eye member 14. In some embodiments, the cylindrical passage 32 defined by the inner surface 34 has a substantially equal diameter throughout a thickness of the eye member 14. In other embodiments, the cylindrical passage 32 has one or more rounded edges and/or has a portion of slightly larger diameter near one or more termini of the cylindrical passage 32. In general, however, the diameter of the cylindrical passage 32 (or of a major portion thereof) is sized to be slightly smaller than a maximum diameter of an orthopedic screw head 36 (not shown in Figure 1, but shown in Figures 9-14) of an orthopedic screw 38, such that the screw head 36 can be forced into the cylindrical passage 32, but only with a sufficiently large force, thereby creating a press fit or interference fit.

As used herein, the terms "press fit" or "interference fit" shall be interpreted broadly as including the joining of any two mating parts such that one or the other (or both) parts slightly deviate in size from their nominal dimension, thereby deforming each part slightly, each being compressed, the interface between the two parts creating a union of extremely high friction. The word interference refers to the fact that one part slightly interferes with the space that the other is occupying in its nominal dimension. According to embodiments of the invention, the difference in sizes between the maximum diameter of the screw head 36 and the diameter of the cylindrical passage 32 is sufficiently large that a force sufficient to overcome the resulting interference fit or press fit between the screw head 36 and the eye member 14 is larger than a force sufficient to pull the screw 38 out of a bone. In some embodiments, such a force is at least approximately 200 pounds (approximately 900 Newtons), though those of ordinary skill in the art will recognize that any desirable force of removal may be achieved by way of materials and relative sizing choices.

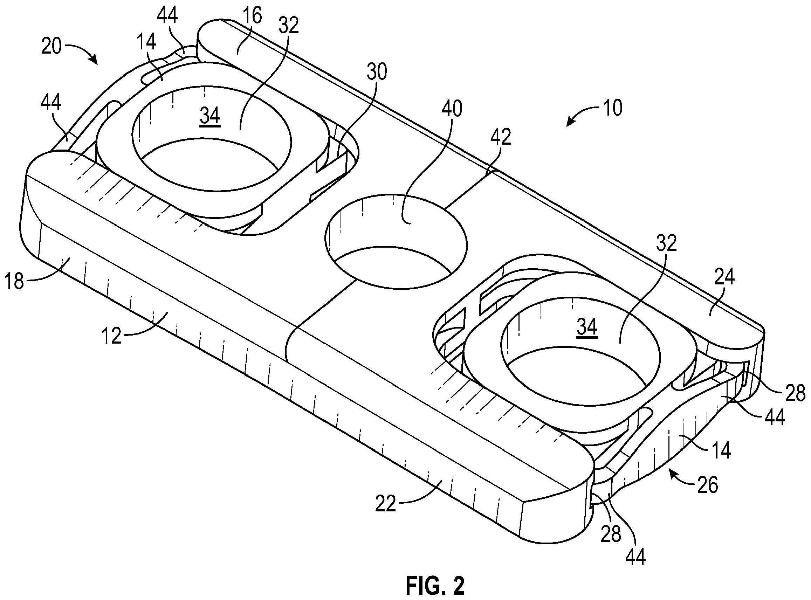

The frame member 12 of Figure 1 also includes a bending interface 40 adapted to receive a tool or part of a tool to facilitate bending of the frame member 12 either prior to implantation or *in situ* after implantation. The bending interface 40 allows the frame member 12 to be bent to better match a contour of the implant location, such as a contour of the anterior portion of the cervical spine. In the embodiment of the frame member 12 shown in Figure 1, the bending interface 40 is centrally located between the first channel 20 and the second channel 26, and the frame member 12 narrows at this location 42. The narrowing of the frame member 12 shown in Figure 1 is an optional feature (compare the embodiment of Figure 2), and whether the frame member 12 is narrower at the location 42 may depend on stresses, aesthetics, or a need for attachment points to the frame member 12, as desired.

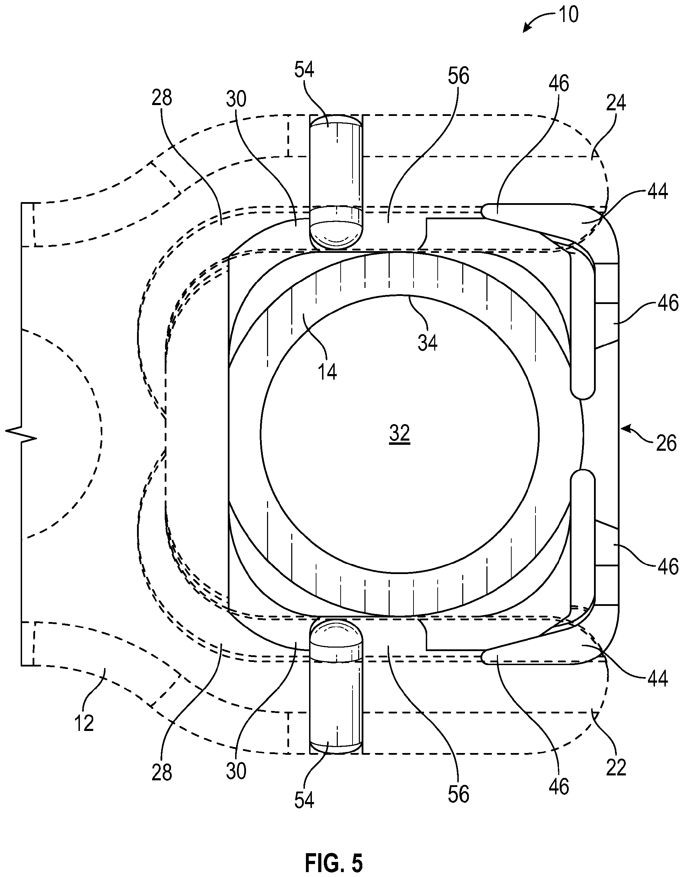

Figure 2 shows an alternate embodiment of the plate 10. As discussed, this plate 10 does not include a narrowing at the location 42. Additionally, in this embodiment, the eye members 14 include a feature or structure that facilitates one-way movement of the eye members 14. In some embodiments, a feature or structure that facilitates one-way movement of the eye members 14 so as to stop or inhibit motion in the opposite direction. Such a feature is intended to allow movement of the eye members 14 toward each other after implantation, such as to account for bone remodeling or subsidence, while still allowing the bone graft or implant to receive compression (either naturally from, e.g., the weight of the head, or aided by a compressive structure such as a spring, a nitinol wire, etc.). In this embodiment the features that selectively inhibits the sliding motion of the eye members 14 within the channels 20, 26 are one-way wedges 44 that are integrated with the eye members 14 (see Figure 5).

As illustrated in Figures 2 and 5, the one-way wedges 44 are formed with the eye members 14 and are shaped to extend into the contours 28 of the legs 16, 18, 22, 24. The wedges 44 of this embodiment are self-energizing wedges that are naturally in tension directed toward a point 46 of each wedge. The tension of each wedge 44 is provided by an arm 46 that connects the wedge 44 to the eye member 14. As the eye member 14 moves inwardly (toward location 42 or, in other words, towards the other eye member 14), the wedge 44 allows the movement, sliding within the contour 28. In contrast, when the eye member 14 attempts to move outwardly (away from location 42 or, in other words, away from the other eye member 14), the wedge 44 is forced between the eye member 14 and the contour 28, thereby impeding the outward motion. Accordingly, the wedge 44 serves to prevent or inhibit outward motion of the eye member 14 and only freely allow inward motion of the eye member 14.

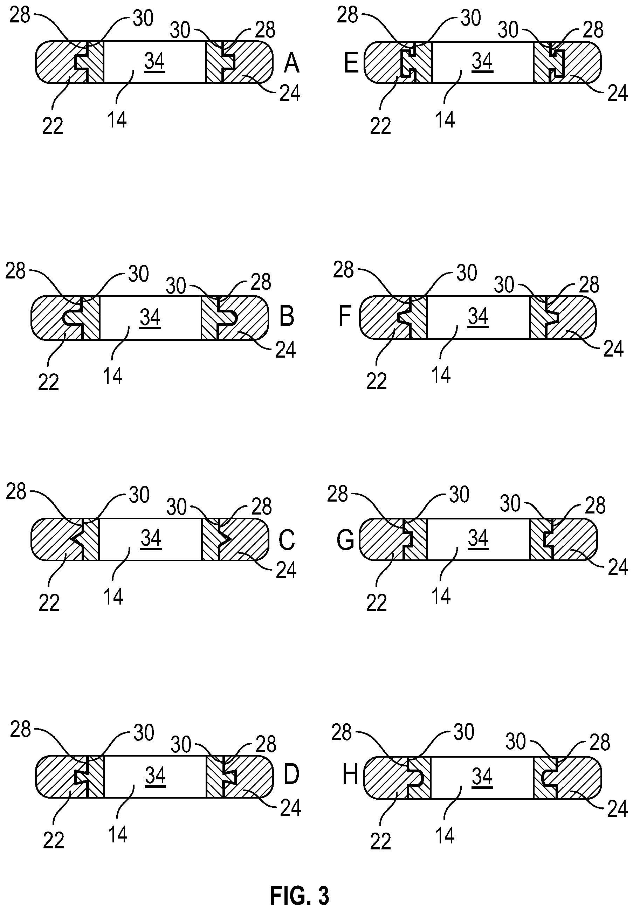

Figure 3 shows cross-sectional views (corresponding to the line 3-3 shown in Figure 1) of various embodiments of the plate 10 showing various embodiments of the contour 28 and the corresponding contour 30 that may exist between the frame member 12 and the eye member 14. While Figure 3 shows various versions of the contour 28 and the contour 30, it should be understood that the versions shown in Figure 3 are only intended to be illustrative, and a variety of alternate embodiments are also embraced within the scope of the invention as disclosed herein, so the specific embodiments illustrated in Figure 3 are not intended to be limiting of the scope of the invention as contained in the claims.

Figure 3 shows cross-sectional views of eight illustrative embodiments labelled A-H. In the embodiment labeled A, the contour 28 forms a slot with a rectangular shape. The rectangular shape may optionally have rounded corners. In this embodiment, the contour 30 forms an extension with a corresponding rectangular shape that also may or may not have rounded corners. In the embodiment labeled B, the contour 28 forms a slot with a semi-circular end, and the contour 30 forms a corresponding extension with a semicircular protruding end. In the embodiment labeled C, the contour 28 forms a slot with a triangular shape, while the contour 30 forms an extension with a corresponding triangular shape. In the embodiment labeled D, the contour 28 forms a slot with a dovetail shape, while the contour 30 forms an extension with a corresponding dovetail shape.

In the embodiment labeled E, the contour 28 forms a slot with out-of-plane protrusions extending upward and downward within the slot, while the contour 30 forms an extension with corresponding out-of-plane extensions to engage the slot's extensions. In the embodiment labeled F, the contour 28 forms a slot with a trapezoidal shape and the contour 30 forms an extension with a corresponding trapezoidal shape. The embodiments labeled G and H illustrate that the contour 30 can form a slot while the contour 28 forms an extension (as opposed to the embodiments labeled A-F). It should be noted that while only two shapes are shown in the embodiments labeled G and H, any shape, including the shapes of the embodiments labeled A-F and other shapes not shown could be provided with an extension on contour 28 and a slot on contour 30. In the embodiment labeled G, the contour 28 forms an extension with a rectangular shape (with or without rounded corners), while the contour 30 forms a slot with a corresponding rectangular shape (with or without rounded corners). In the embodiment labeled H, the contour 28 forms an extension with a semicircular end, and the contour 30 forms a slot with a corresponding semicircular end.

While Figure 3 shows illustrative shapes of slots and extensions forming the contours 28 and 30, it should be understood that any engaging shape of contours 28, 30 may be provided. In some embodiments, each of contours 28 and 30 has one or more corresponding slots and one or more corresponding extension. Those of skill in the art will recognize a variety of shapes that may be provided to contours 28 and 30 so as to provide sliding and secure engagement between the eye member 14 and the frame member 12. Accordingly, such alternate embodiments are embraced as falling within the scope of the claimed invention.

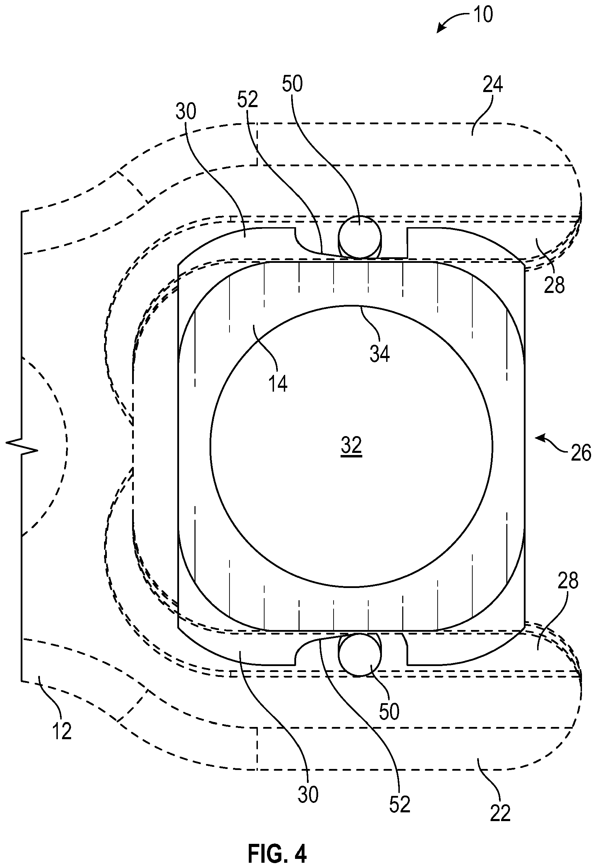

Figure 4 shows a top, partially-transparent view of one end of one embodiment of the plate 10. It will be understood that while Figures 4 and 5 show one end of the plate 10 for purpose of clarity, the illustrated end is representative of features incorporated into the other, not shown end. This view more clearly shows the sliding engagement of the contour 28 and the contour 30 at each of the third leg 22 and the fourth leg 24, so that the eye member 14 can slide inward and outward within the channel 26. The embodiment of Figure 4 also shows another motion-stopping structure adapted to at least selectively inhibit sliding motion of the eye member 14 within the channel 26, in this case a roller clutch. The roller clutch includes a roller 50 disposed between the eye member 14 and the frame member 12 in a beveled slot 52 formed in the contour 30 and within the contour 28. While not shown in Figure 4, the roller 50 may be biased inward by a spring or other energizing structure. As the eye member 14 moves inward, the roller 50 turns freely within the beveled slot 52, but if a force attempts to move the eye member 14 outward, the roller 50 is trapped between the eye member 14 and the frame member 12, thereby inhibiting or stopping the attempted outward motion.

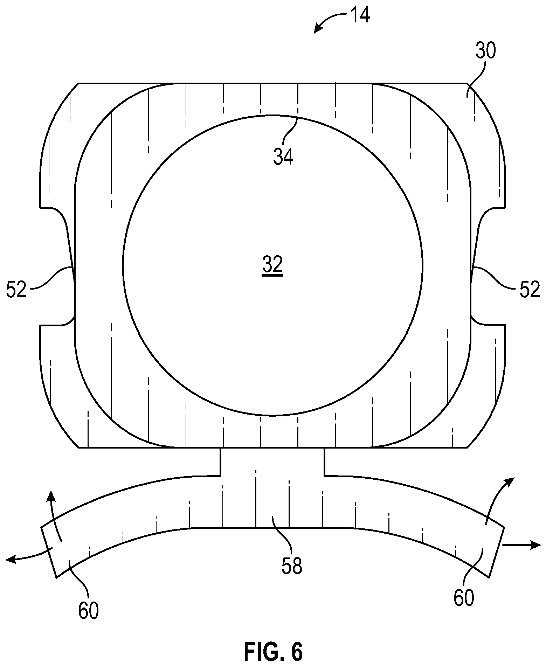

Other structures may be provided to inhibit or stop attempted outward motion, such as a sprag clutch structure, either with or without an energizing structure such as a spring, a ball clutch structure, again either with or without an energizing structure such as a spring, versions of the wedge 44 discussed with respect to Figure 5, a toothed ratchet mechanism, an integrated camming member (see Figure 6), or the like. In some embodiments, a restriction against movement between the eye member 14 and the frame member 12 occurs upon formation of an interference fit between the eye member 14 and the screw head 36. In such embodiments, formation of the interference fit between the eye member 14 and the screw head 36 causes the eye member 14 to expand sufficiently to form a corresponding interference fit between the eye member 14 and the frame member 12 (e.g., between the contour 28 and the contour 30). As will be understood, formation of such an interference fit between the eye member 14 and the frame member 12 will necessarily limit motion in both directions rather than in one direction only.

Figure 5 shows a top, partially-transparent view of one end of another embodiment of the plate 10. This embodiment illustrates the wedge-type restriction of sliding motion to motion in one direction as discussed previously. In addition, however, Figure 5 also illustrates one embodiment of a retention mechanism that retains the eye member 14 within the channel 26 formed by the third leg 22 and the fourth leg 24. In this embodiment, the retention mechanism is a pin 54. The pin 54 is disposed in one or both of the third leg 22 and the fourth leg 24 such that a portion of the pin 54 is disposed within the contour 28. The pin engages a corresponding slot 56 provided in the contour 30 so as to retain the eye member 14 within the channel 26. The engagement of the pin 54 prevents outward motion of the eye member 14 beyond a certain maximal extent, thereby providing an auxiliary mechanism for retaining the eye member 14 within the channel 26.

Figure 6 shows a top view of another embodiment of the eye member 14. This embodiment includes an integrated camming member 58 in the shape of two arms 60. The arms 60 extend from a distal end of the eye member 14 and are shaped such that inward movement of the eye member 14 is freely or relatively freely allowed, but an outward movement of the eye member 14 will be limited. If an outward force is applied to the eye member 14, ends of the arms 60 will engage the contour 28 of the third leg 22 and the fourth leg 24 and limit or prevent outward movement of the eye member 14. As may be seen in Figure 6, multiple motion-limiting structures may be combined, such as the integrated camming member 58 with the beveled slots 52 of a roller clutch discussed with respect to Figure 4.

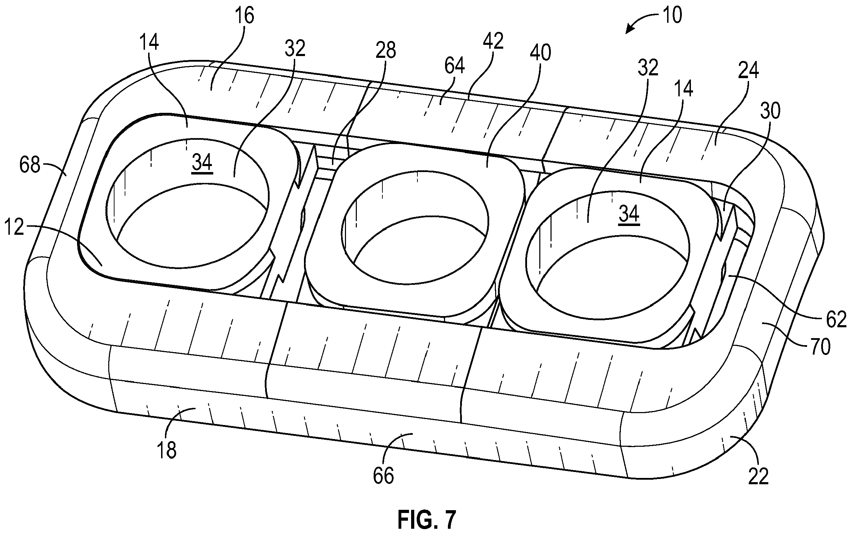

Figure 7 shows a perspective view of an alternate embodiment of the plate 10. In the embodiments of Figures 1-6, the channels 20, 26 were open-ended channels extending from a closed midpoint at the location 42. The embodiment of Figure 7 is different in that there are not two separate channels 20, 26 as in the prior embodiments, but instead a single unitary channel 62 is present. The frame member 12 may still be viewed as having the first leg 16, the second leg 18, the third leg 22, and the fourth leg 24, but in this embodiment, the first leg 16 and the fourth leg 24 are connected by a first central part 64, and the second leg 18 and the third leg 22 are connected by a second central part 66. In this case, the channel 62 is closed-ended on both ends, such that the first leg 16 and the second leg 18 are connected by a first end member 68, and the third leg 22 and the fourth leg 24 are connected by a second end member 70. Thus, in this embodiment, the frame member 12 forms a closed ring enclosing the channel 62. The channel 62 still retains the contour 28, and each of the eye members 14 retain their contours 30.

During manufacture of the embodiment of the plate 10 of Figure 7, the eye members 14 may each be inserted into the channel 62 at the location 42, after which a spacer 72 is inserted at the location 42 to prevent the eye members 14 from leaving the frame member 12. The spacer 72 may be temporarily or permanently affixed at the location 42, and may be formed of a similar material to the frame member 12 and/or the eye members 14, or of a different biocompatible material. In some embodiments, however, the spacer 72 may be removable, such as after implantation of the frame 10, as the eye members 14 will not readily leave the channel 62 when secured to the screw heads 36.

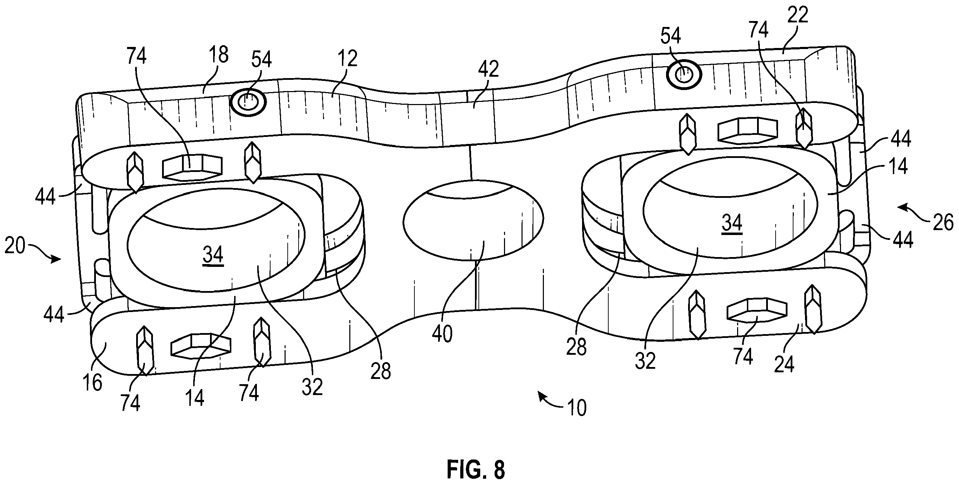

Figure 8 shows a perspective bottom view of another embodiment of the plate 10. In this embodiment, an underside of the plate 10 is shown, being the side that upon implantation will face the bone. In this embodiment, the underside of the plate 10 is provided with one or more teeth, spikes, blades, barbs, or other texture (hereafter teeth 74). The teeth 74 serve to promote torsional stability of the plate 10 upon implantation, especially in cases where the implanted screws 38 are close to parallel. When the screws are not as closely parallel, torsional stability will be more inherent, as the screw will not be able to rotate in the bone without encountering significant resistance from adjacent levels of the spine.

Figure 9 illustrates an alternate embodiment of the plate 10. This Figure is illustrated as the plate 10 would be locked with the screws 38 upon implantation of the plate 10, such as in a cervical spinal fixation procedure. In this embodiment, the plate 10 differs from the previous embodiments in that only a single eye member 14 is present. The other eye member 14 in this embodiment is replaced by a fixed cylindrical passage 76. The passage 76 serves a similar function to and is similarly sized and shaped with the passage 32 of the eye member 14, but lacks the sliding relationship with the frame member 12. In this embodiment, sufficient sliding motion between the cylindrical passage 76 and the cylindrical passage 32 is provided by the first channel 20 and the sliding relationship of the eye member 14 in the first channel 20. Accordingly, the embodiment of Figure 9 shows an alternate manner in which advantages of embodiments of the invention may be achieved.

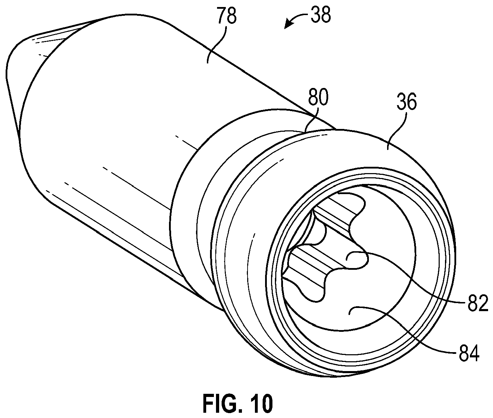

Figure 10 shows a perspective view of one embodiment of the orthopedic screw 38. In this embodiment, a threaded shaft 78 is connected to the screw head 36 by a neck 80 and extends therefrom. The screw head 36 has an outer spherical surface of a diameter that enables the screw head 36 to engage with the inner surface 34 of cylindrical passage 32 or the cylindrical passage 76 at any angle until the neck 80 contacts the edge of the cylindrical passage 32. Accordingly, there are fewer restrictions on the angles between the screws 38 used with the plate 10 than screws used with prior orthopedic plates. The varying orientations possible between the screws 38 is shown, for example, in the illustrated embodiment of Figure 9, which shows how the interference fit between the screw heads 36 and the eye member 14 and the cylindrical passage 76 of the frame member 12 is achieved even though the screws 38 are not parallel to the axis of the cylindrical passage 32 or the cylindrical passage 76.

The screw 38 also includes a driving feature 82. The driving feature 82 of this embodiment is illustrated as a hexalobe internal driving feature, but the screw 38 may be provided with any of a variety of internal and external driving features, and embodiments of the invention are not limited to any particular driving feature of the screw 38. The screw 38 also incorporates a wand attachment feature 84, which in this embodiment is illustrated as an undercut spherical diameter formed in the screw head 36. The screw 36 may be provided with any of a variety of internal attachment points, such as threaded and other undercut shapes, and embodiments of the invention are not limited to any particular engagement feature of the screw 38. The attachment feature 38 serves as an attachment point for a locking wand, but may also serve as a retaining feature for drivers, calipers, distractors, and other instruments.

In the embodiment of Figure 10, the attachment feature 84 serves to allow a wand head having a plurality of fingers to be inserted into the attachment feature 84 while an internal expansion shaft is withdrawn from the wand head. In this state, the fingers are free to flex inwardly so that the wand head can be inserted. Then, the expansion shaft is advanced into the wand head, such that the fingers are impeded from flexing inwardly, and the screw 38 is retained on the wand. In some embodiments, the expansion shaft terminates in a driver that is adapted to engage the driving feature 82, such that the wand may be used to both secure the screw 38 and to drive the screw 38, but such is not required.

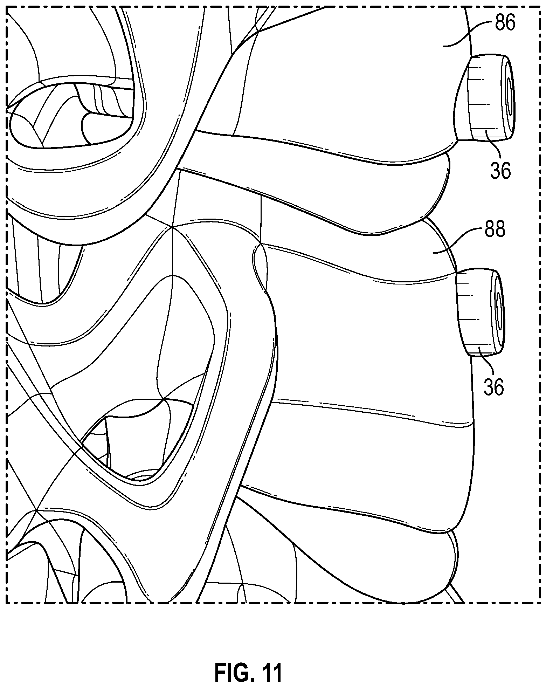

Figures 11-15 illustrate how embodiments of the invention are used in a surgical procedure (e.g., a cervical spine fixation procedure). Figure 11 illustrates how a pair of screws 38 are inserted into bone, e.g., into an anterior portion of a first vertebral body 86 and of a second vertebral body 88, until the screw head 36 is proximate the bone a desired amount (typically flush or nearly flush with the bone). Because of the flexibility with which embodiments of the plate 10 can be attached to the screw heads 36, the surgeon can place the screws 38 into the best bone with the best angle of placement. Additionally, because the screws 38 are placed without any other instrumentation, implant (such as a plate), screw guide, or the like, the surgical wound is essentially "empty" when compared with prior-art methods, and the surgeon is able to fully visualize the surgical site to best place the screws 38 in the bone. Accordingly, the placement of the screws 38 is greatly reduced in difficulty with respect to prior-art methods.

Once the screws 38 have been placed, the screws 38 can be used in a manner similar to which Caspar pins had been used previously to facilitate distraction of the disc space, preparation of the disc/disc space, and placement/insertion of a bone graft or interbody spacer/implant. Thus, according to embodiments of the invention, the surgeon may use a distraction instrument that is inserted into the wand attachment feature of the screws 38 and is used to distract the vertebral bodies. Once the discectomy has been performed along with any other disc space preparation, and once the bone graft or interbody spacer has been placed, the distraction may be released and the distraction instrument or instruments are removed. Because no Caspar pins were used, there is nothing to be removed from the bone itself, and there is no need to perform any treatment to minimize bone bleeding.

Either at this point or previously, the surgeon uses a measurement technique to determine an appropriate spacing between the screw heads 36. The appropriate spacing may involve some measure of distraction, although typically once the graft or interbody implant is in place no additional distraction is required. The measurement technique may be any desirable technique. By way of example, the measurement technique may involve taking a radiograph and measuring a spacing of the screw heads 36 *in situ.* As another example, the spacing of the screw heads 36 *in situ* may occur through use of a caliper or other measurement tool inserted into the wand attachment features 84 of the screw heads 36. As another example, the spacing of the screw heads 36 may be measured by an app running on a computing device (even as simple as a smart phone) using a photograph of the surgical site and the screw heads 36, as a diameter of the screw heads 36 is known. As discussed previously, embodiments of the plate 10 accommodate a variety of screw spacings, so the measurement of screw spacing need not be as precise as with prior-art orthopedic plates, but a generally accurate measurement facilitates selection of a mostappropriately sized plate 10.

In contrast to prior-art methods, the selection of the size of the plate 10 occurs after the screws 38 have been placed. The size of the plate 10 can be selected based on the optimal placement and orientation of the screws 38 after they have already been placed. Prior-art orthopedic plates and techniques required selection of a desired plate size along with use of individual drilling guides and other tools specifically sized for each plate and that limited placement and orientation of the screws. Accordingly, prior-art devices often resulted in lessthan-optimal placement of anchoring screws, which represents a significant risk of adverse results. Embodiments of the invention thus provide significant advantages with respect to screw placement and selection of an optimally sized plate 10.

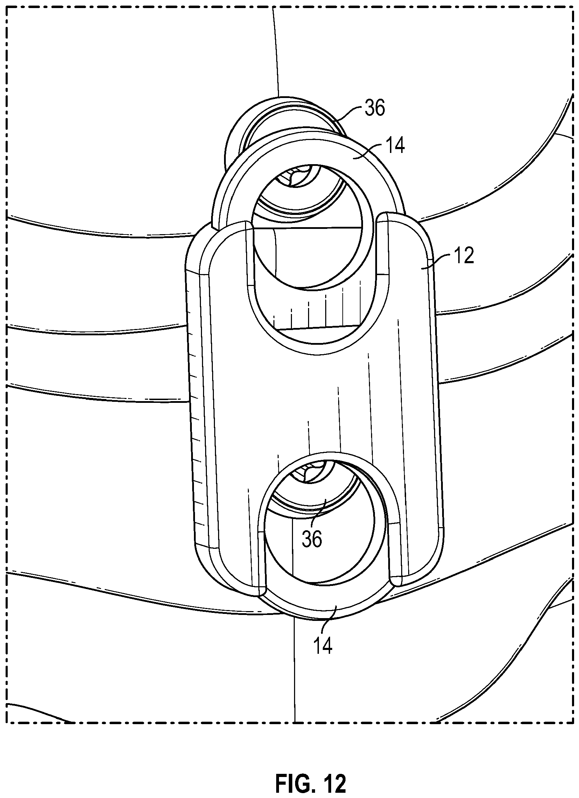

At this point in the procedure, wands are locked into one or more of the wand attachment features 84 of the screw heads 36, and the plate 10 is passed over and down the wands with the wands in the passage(s) 32 and/or passage 76 until the plate 10 approaches or contacts the screw heads 36 as shown in Figure 12 (the wands are not shown) in Figure 12. It should be noted that Figure 12 shows an alternate embodiment of the plate 10. Alternatively, the surgeon places the plate 10 into the surgical wound proximate the screw heads 36 and inserts the wand heads into the wand attachment features 84 through the passage(s) 32 and/or passage 76. At this point, the passage(s) 32 and/or passage 76 are immediately over the screw heads 36 with the eye member(s) 14 and/or frame member 12 surrounding the passage(s) 32 and/or passage 76 in contact with the screw heads 36.

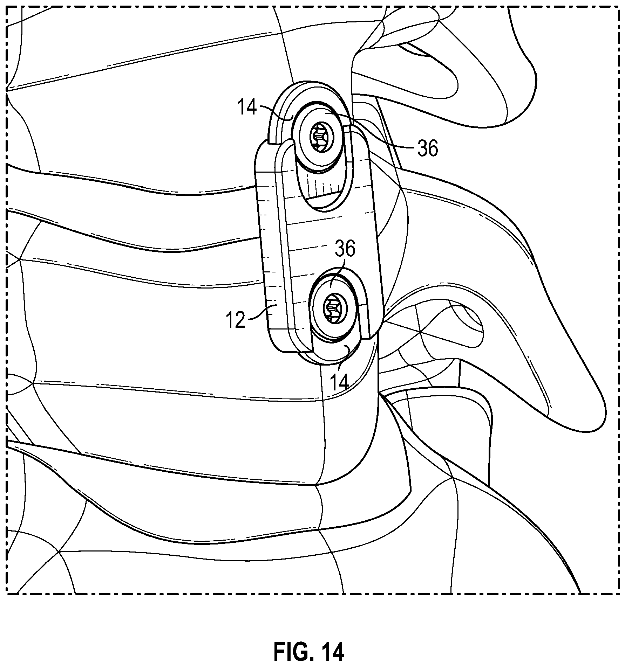

The plate 10 is locked to the screw heads 36 by application of forces between the plate 10 and the screw heads 36 that forces the screw heads 36 into the passage(s) 32 and/or passage 76 until an interference fit is achieved between the screw heads 36 and the eye member(s) 14 and/or frame member 12. The forces for locking each screw 38 to the plate 10 may be applied serially or in parallel. Figure 14 illustrates

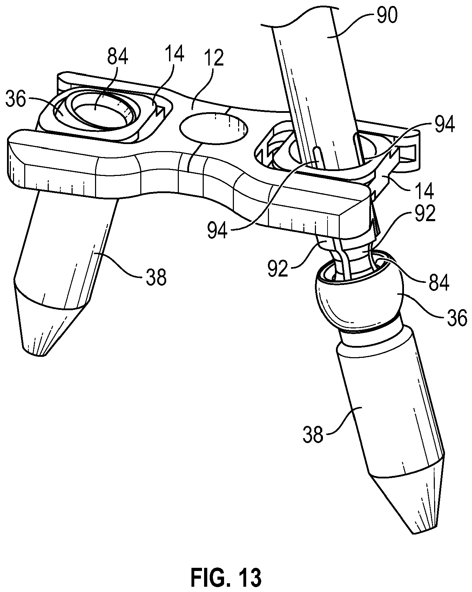

Figure 13 illustrates a perspective view of an embodiment of the plate 10 already fixed to one screw 38 with one embodiment of a wand 90 engaged with the second screw 38 so as to allow application of a force between the screw 38 and the eye member 14. In Figure 14, the distance between the screw head 36 engaged with the wand 90 and the respective eye member 14 has been greatly exaggerated for purposes of illustration, as such a separation would not occur during an actual surgical procedure once the first screw 38 had been locked. As shown in Figure 14, the wand 90 includes a wand head formed of a plurality of fingers 92 separated by slots 94. These fingers 92 allow the wand head to enter into the wand attachment feature 84 by inward flexion of the fingers 92 while an inner expansion shaft (not shown) is withdrawn from the wand head. To lock the screw to the wand head, the inner expansion shaft is advanced until it prevents the fingers from inward flexion, whereupon the wand can be used to apply a force to the screw 38 in a direction generally upward in Figure 13, while a corresponding opposite force is applied to the plate 10 (either at the eye member 14 or at the frame member 12 or both) to cause the screw head 36 to enter the passage 32 and form an interference fit between the screw head 36 and the eye member 14. The screw head 36 is thus effectively drawn into the passage 32.

As may be seen in Figure 13, the wand head may have a generally spherical shape such that the wand head can be disposed in the wand attachment feature 84 with a variety of orientations. This allows the mating forces to be applied in a most advantageous direction during the step of locking the plate 10 to the screws 38, regardless of the orientation of the screw 38. In some embodiments, the force between the plate 10 and the screw head 36 is provided by a single instrument that serves as a locker, having both the wand 90 as well as an element that applies a force to the plate 10. In the event unlocking the plate from the screw head 36 becomes necessary, an unlocker may be used that engages the frame member 12 or the eye member 14 and also the screw head 36 and forces the screw head 36 down and out of the passage 32 or passage 76. As may be appreciated, the locking step (or unlocking step) occurs as a single action without requiring actuation of a separate locking member such as a locking screw as is common with prior-art systems. Accordingly, embodiments of the invention represent a significant improvement in simplicity of the locking phase of a surgical procedure such as a spinal fixation procedure.

Figure 14 shows a perspective view of the embodiment of the plate 10 after it has been locked to the screw heads 36. As may be seen in this view, the frame member 12, the eye members 14, and the screw heads 36 collectively represent a low profile on the surface of the anterior portion of the cervical spine. This low profile is minimized by the lack of additional features such as retaining screws or other devices to retain the screw heads 36 in the eye members 14. As may be seen in Figure 14, upon completion of the locking step, the positions of the two eye members 14 need not be the same: in the procedure shown in Figure 14, the upper eye member 14 is extended significantly more than the lower eye member 14.

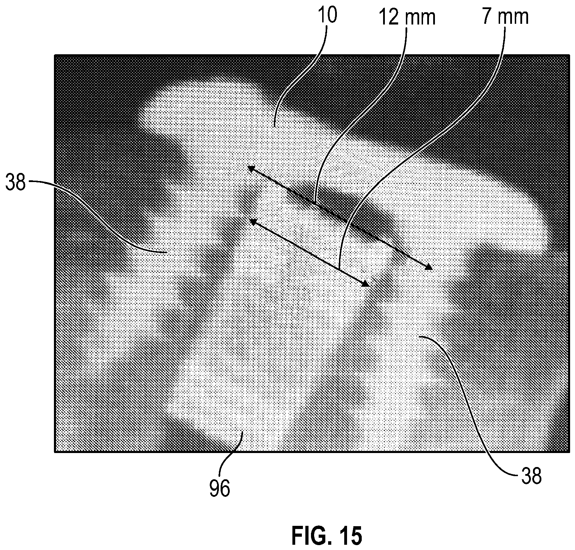

Figure 15 shows one example of a fluoroscopy image of the plate 10 after implantation. While Figure 15 shows an image after a completed procedure, including after insertion of an interbody implant 96 and after the plate 10 has been locked to the two screws 38, Figure 15 also shows how a fluoroscopy image (e.g., an X-ray image) can be used to determine a distance between the two screws 38 so as to permit selection of a proper size for the plate 10. As the sizes of the screw heads 36 are known, the screw heads can be used in a fluoroscopy image (or even in a visual image taken using a conventional visible-light camera) to determine the distance between the screw heads 36, using the screw heads 36 as a reference guide to determine the screw head spacing.

In summary, embodiments of the invention provide many advantages over prior-art orthopedic plates. The embodiments of the plate 10 allow screws to be placed with full visualization. The plate 10 is guided onto the screws 38 by wands 90 without need for locating instruments or pins. The embodiments of the plate 10 allow eye members 14 to translate within the frame members 12 to match the plate size to the screw placement, without requiring an exact plate size match. Accordingly, inventory carrying requirements can be reduced.

Embodiments of the invention obviate the need to achieve a particular position and angulation of the screws 38. The screws 38 allow more angulation than prior-art orthopedic plates, and the eye members 14 of the plate 10 adjust to the screw position. Accordingly, cumbersome drill and screw guides that were needed previously to place and angle screws correctively relative to the prior-art plates are no longer needed. Furthermore, embodiments of the new plate 10 are introduced into the surgical wound after the screws 38 are placed, so the wound is comparatively empty during screw placement, whereas prior-art orthopedic plates required the plate to be present in the wound during screw placement, interfering with the visualization needed to achieve proper screw placement.

Embodiments of the plate 10 allow the eye members 14 to slide to maintain graft contact. Optional unidirectional regulation of movement of the eye members 14 of certain embodiments assists in maintaining graft compression. Prior-art orthopedic plates, however, typically did not adjust to bone remodeling or subsidence. Embodiments of the plate 10 also allow the implanted screws 38 to function as attachment points for other instruments such as distraction instruments, so separate Caspar pins are not required for distraction, as was often required with prior-art systems. Accordingly, there is less injury done to surrounding bone that must be treated.

Many prior-art orthopedic plates require separate screw-plate retention mechanisms that add bulk to the systems, increase cost of manufacture of the systems, add surgical steps, and that may restrict screw angulation during placement. Embodiments of the plate 10 address these deficiencies. The eye members 14 lock directly onto the screw heads 36 by an interference or press fit and do not require the typical prior-art ring, propeller or wire to sit above the screw head. As a result, the thickness of the system is reduced. The embodiments of the plate 10 also have a reduced locking-related parts count because the retention of the screws 38 is inherent to the eye members 14 rather than secondary. Surgical steps are reduced, as the embodiments of the plate 10 obviate the need for actuating a locking mechanism. Finally, as retention of the screw heads 36 in the plate 10 is inherent and independent of screw angulation up to the point of the screw neck 80 contacting the eye members 14, a wide range of angulation of the screws 38 is permitted.

Surgical techniques associated with embodiments of the plate 10 are significantly simplified with respect to surgical techniques utilized with prior-art systems. A prior-art surgical technique, after creation of the surgical wound to expose the anterior cervical spine would typically involve a number of steps. Such steps would include inserting Caspar-style pins into the vertebral bodies, followed by use of a distractor attached to the pins to distract the disc space. A discectomy would be performed, followed by insertion of an interbody spacer and/or graft. Then, a plate would be trialed in the wound to determine if the plate was appropriately sized. In some instances, multiple plates would be trialed (at an increased cost in sanitation requirements and/or plates considered used and to be destroyed after the surgery). Once a plate size is determined, the plate is positioned and a screw hole is predrilled. The first screw is inserted, the plate is recentered, and the second hole is predrilled and the second screw is inserted. The third and fourth screws are then predrilled and inserted. Typically, a screw-locking procedure is followed for each screw. Thereafter, the Caspar-style pins are removed and bone wax is used to control bleeding.

In some alternate prior-art orthopedic plate insertion procedures, the trialing steps are performed using drill/screw guides. With such systems, a drill guide is positioned after which the first screw hole is pre-drilled, followed by predrilling of the other three screw holes. Care must be taken not to allow the drill guide to move during this procedure, which could cause misalignment such that the plate would not fit properly. Then, the plate is positioned in the wound, and the screws are inserted. Again, a screw-locking procedure is followed for each screw. The steps of removing the Caspar-style pins and using bone wax to control bleeding are similar.

In contrast, the surgical procedure for use with embodiments of the plate 10 is simplified. No Caspar-style pins are used. Instead, the procedure begins, after creation of the surgical wound to expose the anterior cervical spine, with insertion of the two screws 38 into the vertebral bodies with full visualization and with allowance for a greater degree of flexibility in screw placement and angulation. The screws 38 themselves then serve as attachment points for the distractor, and distraction follows with the discectomy and insertion of the interbody spacer and/or graft. The plate length is estimated off of an image (fluoroscopy and/or visual imagery), the plate 10 is inserted into the wound, and the plate 10 is locked to the screw heads 36, whereupon the procedure is done. No separate screw-locking procedure is necessary, and there are no Caspar-style pin wounds to be treated.

While embodiments of the invention have been described herein, it is envisioned that alternate embodiments may also be provided. In one alternate style of the plate 10, the plate 10 is formed of two integrated frame/eye members that incorporate mutual sliding features whereby they slide with respect to each other and not as two eye members 14 sliding with respect to a separate frame member 12 as with certain embodiments previously discussed. The one-eyemember embodiment discussed with respect to Figure 9 is just one example of a design with only two members sliding with respect to one another without sliding interaction with a third member.

The described embodiments are to be considered in all respects only as illustrative and not restrictive. The scope of the invention is, therefore, indicated by the appended claims, rather than by the foregoing description.

## Claims

1. An orthopedic plate and screw system, comprising:
an orthopedic screw (38) having threaded shaft (78) extending from an orthopedic screw head (36) having an outer spherical surface;
an eye member (14) comprising:
a biocompatible material formed to define a cylindrical passage (32) through the eye member (14); and
a first frame member engagement contour (30) and a second frame member engagement contour (30) formed on opposite sides of the eye member (14); and
a frame member (12) comprising:
a channel (20) formed by a first frame leg (16) and a second frame leg (18), both of a biocompatible material;
the first frame leg (16) comprising a first eye member engagement contour (28) formed on a channel side of the first fame leg (16) and adapted to engage the first frame member engagement contour (30) of the eye member (14) in sliding engagement; and
the second frame leg (18) comprising a second eye member engagement contour (28) formed on a channel side of the second fame leg (18) and adapted to engage the second frame member engagement contour (30) of the eye member (14) in sliding engagement;
wherein the eye member (14) is contained within and adapted to slide within the channel (20), **characterised in that**
the cylindrical passage (32) is sized slightly smaller than the orthopedic screw head (36) whereby the cylindrical passage (32) is adapted to receive head-first introduction of the orthopedic screw head (36) therein and to provide an interference fit of the eye member (14) with the orthopedic screw head (36).

2. The orthopedic plate and screw system of claim 1, wherein the first frame member engagement contour (30) and the second frame member engagement contour (30) each comprise an extension extending laterally away from a center of the eye member (14), and wherein the first eye member engagement contour (28) and the second eye member engagement contour (28) each comprise a slot sized and shaped to slidingly receive one extension of the eye member (14).

3. The orthopedic plate and screw system of claim 2, wherein the extension of the first frame member engagement contour (30) and the second frame member engagement contour (30) comprises a cross-sectional shape selected from the group consisting of:
a rectangular shape;
a rectangular shape with rounded corners;
a dovetail shape;
a partially circular shape;
a partially ellipse shape;
a triangular shape;
a trapezoidal shape;
a polygonal shape; and
a shape with one or more out-of-plane protrusions.

4. The orthopedic plate and screw system of claim 1, wherein the first eye member engagement contour (28) and the second eye member engagement contour (28) each comprise an extension extending laterally toward the other frame leg (16, 18), and wherein the first frame member engagement contour (30) and the second frame member engagement contour (30) each comprise a slot sized and shaped to slidingly receive one extension of the frame member (12).

5. The orthopedic plate and screw system of claim 4, wherein the extension of the first eye member engagement contour (28) and the second eye member engagement contour (28) comprises a cross-sectional shape selected from the group consisting of:
a rectangular shape;
a rectangular shape with rounded corners;
a dovetail shape;
a partially circular shape;
a partially ellipse shape;
a triangular shape;
a trapezoidal shape;
a polygonal shape; and
a shape with one or more out-of-plane protrusions.

6. The orthopedic plate and screw system of claim 1, wherein the frame member further comprises a static cylindrical passage (76) sized to receive head-first introduction of another orthopedic screw head (36) of another orthopedic screw (38) therein and to provide an interference fit with the other orthopedic screw head (36).

7. The orthopedic plate and screw system of claim 1, further comprising another eye member (14), wherein:
the other eye member (14) comprises:
a biocompatible material formed to define a cylindrical passage (32) through the other eye member (14), the cylindrical passage (32) through the other eye member (14) being sized to receive head-first introduction of another orthopedic screw head (36) of another orthopedic screw (38) therein and to provide an interference fit with the other orthopedic screw head (36); and
a third frame member engagement contour (30) and a fourth frame member engagement contour (30) formed on opposite sides of the other eye member (14); and
the frame member (12) further comprises another channel (26) formed by a third frame leg (22) and a fourth frame leg (24), both of a biocompatible material;
the third frame leg (22) comprising a third eye member engagement contour (28) formed on a channel side of the third fame leg (22) and adapted to engage the third frame member engagement contour (30) of the other eye member (14) in sliding engagement; and
the fourth frame leg (24) comprising a fourth eye member engagement contour (28) formed on a channel side of the fourth fame leg (24) and adapted to engage the fourth frame member engagement contour (30 of the other eye member (14) in sliding engagement;
wherein the other eye member (14) is contained within and adapted to slide within the other channel (26).

8. The orthopedic plate and screw system of claim 1, further comprising another eye member (14), wherein:
the other eye member (14) comprises:
a biocompatible material formed to define a cylindrical passage (32) through the other eye member (14), the cylindrical passage (32) through the other eye member being sized to receive head-first introduction of another orthopedic screw head (36) of another orthopedic screw (38) therein and to provide an interference fit with the other orthopedic screw head (36); and
a third frame member engagement contour (30) and a fourth frame member engagement contour (30) formed on opposite sides of the other eye member (14) and adapted to engage the first and second eye member engagement contours (28) of the first and second frame legs (16, 18); and
the other eye member (14) is contained within and adapted to slide within the channel (20).

9. The orthopedic plate and screw system of claim 1, wherein the orthopedic screw (38) comprises a driving feature (82) and a wand-attachment feature (84) adapted to permit retention of the orthopedic screw (38) against a force directed against the orthopedic screw head (36) during interference fitting of the orthopedic screw head (36) in the cylindrical passage (32).

10. The orthopedic plate and screw system of claim 1, further comprising a motion-stopping structure adapted to at least selectively inhibit sliding motion of the eye member (14) within the channel (20).

11. The orthopedic plate and screw system of claim 10, wherein the motion-stopping structure comprises a structure selected from the group consisting of:
a cylindrical roller clutch structure;
a sprag clutch structure;
a ball clutch structure;
a cylindrical roller clutch structure with an energizing spring;
a sprag clutch structure with an energizing spring;
a ball clutch structure with an energizing spring;
a toothed ratchet mechanism;
a self-energizing wedge;
an integrated camming member; and
an interference fit between the eye member (14) and the channel (20) caused by expansion of the eye member (14) upon insertion of the orthopedic screw head (36) into the cylindrical passage (32).

12. The orthopedic plate and screw system of claim 1, wherein the channel (20) has a shape selected from the group consisting of an open-ended shape and a closed-ended shape.

13. The orthopedic plate and screw system of claim 1, wherein the orthopedic plate (10) is sized for use in a cervical vertebra fixation procedure.

14. The orthopedic plate and screw system of claim 1, wherein the system comprises:
the orthopedic screw (38) being a first orthopedic screw (38) having a first threaded shaft (78) extending from a first orthopedic screw head (36);
a second orthopedic screw (38) having a second threaded shaft (78) extending from a second orthopedic screw head (36) having an outer spherical surface;
the eye member (14) being a first eye member (14)
a second eye member (14) comprising:
a biocompatible material formed to define a second cylindrical passage (32) through the second eye member (14), the second cylindrical passage (32) through the second eye member (14) being sized slightly smaller than the orthopedic screw head (36) whereby the cylindrical passage (32) is adapted to receive head-first introduction of the second orthopedic screw head (36) therein and to provide an interference fit of the second eye member (14) with the second orthopedic screw head (36); and
a third frame member engagement contour (30) and a fourth frame member engagement contour (30 formed on opposite sides of the second eye member (14); and
the channel (26) of the frame member being a first channel (26) and the frame member further comprising:
a second channel (26) formed by a third frame leg (22) and a fourth frame leg (24), both of a biocompatible material;
the third frame leg (22) comprising a third eye member engagement contour (28) formed on a channel side of the third fame leg (22) and adapted to engage the third frame member engagement contour (30) of the second eye member (14) in sliding engagement; and
the fourth frame leg (24) comprising a fourth eye member engagement contour (28) formed on a channel side of the forth fame leg (24) and adapted to engage the fourth frame member engagement contour (30) of the second eye member (14) in sliding engagement;
wherein the second eye member (14) is contained within and adapted to slide within the second channel (26).

15. The orthopedic plate and screw system of claim 14, further comprising a force-generating mechanism adapted to apply a compressive force between the first eye member (14) and the second eye member (14).

## Patentansprüche

1. Orthopädisches Platten- und Schraubensystem, umfassend:
eine orthopädische Schraube (38) mit einem Gewindeschaft (78), der sich von einem orthopädischen Schraubenkopf (36) mit einer kugelförmigen Außenseite erstreckt;
ein Ösenelement (14) umfassend:
ein biokompatibles Material, das zu ausgebildet ist, einen zylindrischen Kanal (32) durch das Ösenelement (14) zu begrenzen;
eine Eingriffskontur (30) eines ersten Rahmenelements und eine Eingriffskontur (30) eines zweiten Rahmenelements, die auf gegenüberliegenden Seiten des Ösenelements (14) ausgebildet sind; und
ein Rahmenelement (12) umfassend:
einen Kanal (20), der durch einen ersten Rahmenschenkel (16) und einen zweiten Rahmenschenkel (18) gebildet wird, die beide aus einem biokompatiblen Material bestehen;
wobei der erste Rahmenschenkel (16) eine Eingriffskontur (28) eines ersten Ösenelements umfasst, die auf einer Kanalseite des ersten Rahmenschenkels (16) ausgebildet ist und dazu ausgelegt ist, mit der Eingriffskontur (30) des Ösenelements (14) des ersten Rahmenelements in Gleiteingriff zu kommen; und
wobei der zweite Rahmenschenkel (18) eine Eingriffskontur (28) eines zweiten Ösenelements umfasst, die auf einer Kanalseite des zweiten Rahmenschenkels (18) ausgebildet ist und dazu ausgelegt ist, mit der Eingriffskontur (30) des Ösenelements (14) des zweiten Rahmenelements in Gleiteingriff zu kommen;
wobei sich das Ösenelement (14) in dem Kanal (20) befindet und darin gleiten kann, **dadurch gekennzeichnet, dass** der zylindrische Kanal (32) etwas kleiner dimensioniert ist als der orthopädische Schraubenkopf (36), wodurch der zylindrische Kanal (32) dazu ausgelegt ist, den orthopädischen Schraubenkopf (36) mit dem Kopf voraus darin aufzunehmen und eine Presspassung des Ösenelements (14) mit dem orthopädischen Schraubenkopf (36) bereitzustellen.

2. Orthopädisches Platten- und Schraubensystem nach Anspruch 1, wobei die Eingriffskontur (30) des ersten Rahmenelements und die Eingriffskontur (30) des zweiten Rahmenelements jeweils einen Fortsatz umfassen, der sich seitlich weg von einem Mittelpunkt des Ösenelements (14) erstreckt, und wobei die Eingriffskontur (28) des ersten Ösenelements und die Eingriffskontur (28) des zweiten Ösenelements jeweils einen Schlitz umfassen, der so dimensioniert und geformt ist, dass er einen Fortsatz des Ösenelements (14) gleitend aufnehmen kann.

3. Orthopädisches Platten- und Schraubensystem nach Anspruch 2, wobei der Fortsatz der Eingriffskontur (30) des ersten Rahmenelements und der Eingriffskontur (30) des zweiten Rahmenelements eine Querschnittsform umfasst, die ausgewählt ist aus der Gruppe bestehend aus:
einer rechteckigen Form;
einer rechteckigen Form mit gerundeten Ecken;
einer Schwalbenschwanzform;
einer teilweise kreisrunden Form;
einer teilweise elliptischen Form;
einer dreieckigen Form;
einer Trapezform;
einer polygonalen Form; und
einer Form mit einem oder mehreren aus der Ebene ragenden Vorsprüngen.

4. Orthopädisches Platten- und Schraubensystem nach Anspruch 1, wobei die Eingriffskontur (28) des ersten Ösenelements und die Eingriffskontur (28) des zweiten Ösenelements jeweils einen Fortsatz umfassen, der sich seitlich in Richtung zu dem anderen Rahmenschenkel (16, 18) erstreckt, und wobei die Eingriffskontur (30) des ersten Rahmenelements und die Eingriffskontur (30) des zweiten Rahmenelements jeweils einen Schlitz umfassen, der so dimensioniert und geformt ist, dass er einen Fortsatz des Rahmenelements (12) gleitend aufnehmen kann.

5. Orthopädisches Platten- und Schraubensystem nach Anspruch 4, wobei der Fortsatz der Eingriffskontur (28) des ersten Ösenelements und der Eingriffskontur (28) des zweiten Ösenelements eine Querschnittsform umfasst, die ausgewählt ist aus der Gruppe bestehend aus:
einer rechteckigen Form;
einer rechteckigen Form mit gerundeten Ecken;
einer Schwalbenschwanzform;
einer teilweise kreisrunden Form;
einer teilweise elliptischen Form;
einer dreieckigen Form;
einer Trapezform;
einer polygonalen Form; und
einer Form mit einem oder mehreren aus der Ebene ragenden Vorsprüngen.

6. Orthopädisches Platten- und Schraubensystem nach Anspruch 1, wobei das Rahmenelement ferner einen statischen zylindrischen Kanal (76) umfasst, der so dimensioniert ist, dass er einen weiteren orthopädischen Schraubenkopf (36) einer weiteren orthopädischen Schraube mit dem Kopf voraus darin aufnehmen kann und eine Presspassung mit dem weiteren orthopädischen Schraubenkopf (36) bereitstellen kann.

7. Orthopädisches Platten- und Schraubensystem nach Anspruch 1, ferner umfassend ein weiteres Ösenelement (14), wobei:
das weitere Ösenelement (14) Folgendes umfasst:
ein biokompatibles Material, das dazu ausgebildet ist, einen zylindrischen Kanal (32) durch das weitere Ösenelement (14) zu begrenzen, wobei der zylindrische Kanal (32) durch das weitere Ösenelement (14) so dimensioniert ist, dass er einen weiteren orthopädischen Schraubenkopf (36) einer weiteren orthopädischen Schraube (38) mit dem Kopf voraus darin aufnehmen kann und
eine Presspassung mit dem weiteren orthopädischen Schraubenkopf (36) bereitstellen kann; und
eine Eingriffskontur (30) eines dritten Rahmenelements und eine Eingriffskontur (30) eines vierten Rahmenelements, die auf gegenüberliegenden Seiten des weiteren Ösenelements (14) ausgebildet sind; und
wobei das Rahmenelement (12) ferner einen weiteren Kanal (26) umfasst, der durch einen dritten Rahmenschenkel (22) und einen vierten Rahmenschenkel (24) gebildet wird, die beide aus einem biokompatiblen Material bestehen;
wobei der dritte Rahmenschenkel (22) eine Eingriffskontur (28) eines dritten Ösenelements umfasst, die auf einer Kanalseite des dritten Rahmenschenkels (22) ausgebildet ist und dazu ausgelegt ist, mit der Eingriffskontur (30) des weiteren Ösenelements (14) des dritten Rahmenelements in Gleiteingriff zu kommen; und
wobei der vierte Rahmenschenkel (24) eine Eingriffskontur (28) eines vierten Ösenelements umfasst, die auf einer Kanalseite des vierten Rahmenschenkels (24) ausgebildet ist und dazu ausgelegt ist, mit der Eingriffskontur (30) des weiteren Ösenelements (14) des vierten Rahmenelements in Gleiteingriff zu kommen;
wobei sich das weitere Ösenelement (14) in dem weiteren Kanal (26) befindet und darin gleiten kann.

8. Orthopädisches Platten -und Schraubensystem nach Anspruch 1, ferner umfassend ein weiteres Ösenelement (14), wobei
das weitere Ösenelement (14) Folgendes umfasst:
ein biokompatibles Material, das dazu ausgebildet ist, einen zylindrischen Kanal (32) durch das weitere Ösenelement (14) zu begrenzen, wobei der zylindrische Kanal (32) durch das weitere Ösenelement (14) so dimensioniert ist, dass er einen weiteren orthopädischen Schraubenkopf (36) einer weiteren orthopädischen Schraube (38) mit dem Kopf voraus darin aufnehmen kann und eine Presspassung mit dem weiteren orthopädischen Schraubenkopf (36) bereitstellen kann; und
eine Eingriffskontur (30) eines dritten Rahmenelements und eine Eingriffskontur (30) eines vierten Rahmenelements, die auf gegenüberliegenden Seiten des weiteren Ösenelements (14) ausgebildet sind und dazu ausgelegt sind, in die Eingriffskonturen (28) des ersten und zweiten Ösenelements des ersten und zweiten Rahmenschenkels (16, 18) einzugreifen; und
das weitere Ösenelement (14) sich in dem Kanal (20) befindet und darin gleiten kann.

9. Orthopädisches Platten- und Schraubensystem nach Anspruch 1, wobei die orthopädische Schraube (38) ein Mitnahmemerkmal (82) und ein Wandbefestigungsmerkmal (84) umfasst, das dazu ausgelegt ist, die Halterung der orthopädischen Schraube (38) gegen eine gegen den orthopädischen Schraubenkopf (36) gerichtete Kraft zu erlauben, während der orthopädische Schraubenkopf (36) mit Presspassung in dem zylindrischen Kanal (32) sitzt.

10. Orthopädisches Platten- und Schraubensystem nach Anspruch 1, ferner mit einer die Bewegung stoppenden Struktur, die dazu ausgelegt ist, eine Gleitbewegung des Ösenelements (14) in dem Kanal (20) wenigstens wahlweise zu unterbinden.

11. Orthopädisches Platten- und Schraubensystem nach Anspruch 10, wobei die die Bewegung stoppende Struktur eine Struktur umfasst, die ausgewählt ist aus der Gruppe bestehend aus:
einer zylindrischen Rollenkupplungsstruktur;
einer Freilaufkupplungsstruktur;
einer Kugelkupplungsstruktur;
einer zylindrischen Rollenkupplungsstruktur mit einer Aktivierungsfeder;
einer Freilaufkupplungsstruktur mit einer Aktivierungsfeder;
einer Kugelkupplungsstruktur mit einer Aktivierungsfeder;
einem verzahnten Ratschenmechanismus;
einem selbstverstärkenden Keil;
einem integrierten Nockenelement; und
einer Presspassung zwischen dem Ösenelement (14) und dem Kanal (20), die durch die Ausdehnung des Ösenelements (14) verursacht wird, wenn der orthopädische Schraubenkopf (36) in den zylindrischen Kanal (32) eingesetzt wird.

12. Orthopädisches Platten- und Schraubensystem nach Anspruch 1, wobei der Kanal (20) eine Form besitzt, die aus der aus einer Form mit offenem Ende und einer Form mit geschlossenem Ende bestehenden Gruppe ausgewählt ist.

13. Orthopädisches Platten- und Schraubensystem nach Anspruch 1, wobei die orthopädische Platte (10) zur Verwendung bei einem Halswirbelfixierungsverfahren dimensioniert ist.

14. Orthopädisches Platten- und Schraubensystem nach Anspruch 1, wobei das System Folgendes umfasst:
die orthopädische Schraube (38), die eine erste orthopädische Schraube (38) mit einem ersten Gewindeschaft (78) darstellt, der sich von einem ersten orthopädischen Schraubenkopf (36) erstreckt;
eine zweite orthopädische Schraube (38) mit einem zweiten Gewindeschaft (78), der sich von einem zweiten orthopädischen Schraubenkopf (36) mit einer kugelförmigen Außenseite erstreckt;
das Ösenelement (14), das ein erstes Ösenelement (14) darstellt;
ein zweites Ösenelement (14), das Folgendes umfasst:
ein biokompatibles Material, das dazu ausgebildet ist, einen zweiten zylindrischen Kanal (32) durch das zweite Ösenelement (14) zu begrenzen, wobei der zweite zylindrische Kanal (32) durch das zweite Ösenelement (14) etwas kleiner dimensioniert ist als der orthopädische Schraubenkopf (36), wodurch der zylindrische Kanal (32) dazu ausgelegt ist, den zweiten orthopädischen Schraubenkopf (36) mit dem Kopf voraus darin aufzunehmen und eine Presspassung des zweiten Ösenelements (14) mit dem zweiten orthopädischen Schraubenkopf (36) bereitzustellen; und
eine Eingriffskontur (30) eines dritten Rahmenelements und eine Eingriffskontur (30) eines vierten Rahmenelements, die auf gegenüberliegenden Seiten des zweiten Ösenelements (14) ausgebildet sind; und
wobei der Kanal (26) des Rahmenelements einen ersten Kanal (26) darstellt und das Rahmenelement ferner Folgendes umfasst:
einen zweiten Kanal (26), der durch einen dritten Rahmenschenkel (22) und einen vierten Rahmenschenkel (24) gebildet wird, die beide aus einem biokompatiblen Material bestehen;
wobei der dritte Rahmenschenkel (22) eine Eingriffskontur (28) eines dritten Ösenelements umfasst, die auf einer Kanalseite des dritten Rahmenschenkels (22) ausgebildet ist und dazu ausgelegt ist, mit der Eingriffskontur (30) des zweiten Ösenelements (14) des dritten Rahmenelements in Gleiteingriff zu kommen; und
wobei der vierte Rahmenschenkel (24) eine Eingriffskontur (28) eines vierten Ösenelements umfasst, die auf einer Kanalseite des vierten Rahmenschenkels (24) ausgebildet ist und dazu ausgelegt ist, mit der Eingriffskontur (30) des zweiten Ösenelements (14) des vierten Rahmenelements in Gleiteingriff zu kommen;
wobei sich das zweite Ösenelement (14) in dem zweiten Kanal (26) befindet und darin gleiten kann.

15. Orthopädisches Platten- und Schraubensystem nach Anspruch 14, ferner umfassend einen Krafterzeugungsmechanismus, der dazu ausgelegt ist, eine Kompressionskraft zwischen dem ersten Ösenelement (14) und dem zweiten Ösenelement (14) aufzubringen.

## Revendications

1. - Système de plaque et de vis orthopédique, comprenant :
une vis orthopédique (38) ayant une tige filetée (78) s'étendant à partir d'une tête de vis orthopédique (36) ayant une surface sphérique extérieure ;
un élément œil (14) comprenant :
un matériau biocompatible formé pour définir un passage cylindrique (32) à travers l'élément œil (14) ;
un premier contour d'engagement d'élément cadre (30) et un deuxième contour d'engagement d'élément cadre (30) formés sur des côtés opposés de l'élément œil (14) ; et
un élément cadre (12) comprenant :
un canal (20) formé par une première branche de cadre (16) et une deuxième branche de cadre (18), toutes deux faites d'un matériau biocompatible ;
la première branche de cadre (16) comprenant un premier contour d'engagement d'élément œil (28) formé sur un côté canal de la première branche de cadre (16) et apte à engager le premier contour d'engagement d'élément cadre (30) de l'élément œil (14) en engagement coulissant ; et
la deuxième branche de cadre (18) comprenant un deuxième contour d'engagement d'élément œil (28) formé sur un côté canal de la deuxième branche de cadre (18) et apte à engager le deuxième contour d'engagement d'élément cadre (30) de l'élément œil (14) en engagement coulissant ; l'élément œil (14) étant contenu à l'intérieur du canal (20) et apte à coulisser à l'intérieur de celui-ci, **caractérisé par le fait que** le passage cylindrique (32) est dimensionné légèrement plus petit que la tête de vis orthopédique (36), ce par quoi le passage cylindrique (32) est apte à recevoir l'introduction tête la première de la tête de vis orthopédique (36) à l'intérieur de celui-ci et à assurer un ajustement serré de l'élément œil (14) avec la tête de vis orthopédique (36).

2. - Système de plaque et de vis orthopédique selon la revendication 1, dans lequel le premier contour d'engagement d'élément cadre (30) et le deuxième contour d'engagement d'élément cadre (30) comprennent chacun une extension s'étendant latéralement à l'opposé d'un centre de l'élément œil (14), et le premier contour d'engagement d'élément œil (28) et le deuxième contour d'engagement d'élément œil (28) comprenant chacun une fente dimensionnée et conformée pour recevoir de manière coulissante une extension de l'élément œil (14).

3. - Système de plaque et de vis orthopédique selon la revendication 2, dans lequel l'extension du premier contour d'engagement d'élément cadre (30) et du deuxième contour d'engagement d'élément cadre (30) comprend une forme en section transversale choisie dans le groupe constitué par :
une forme rectangulaire ;
une forme rectangulaire avec des coins arrondis ;
une forme de queue d'aronde ;
une forme partiellement circulaire ;
une forme partiellement elliptique ;
une forme triangulaire ;
une forme trapézoïdale ;
une forme polygonale ; et
une forme avec une ou plusieurs saillies hors plan.

4. - Système de plaque et de vis orthopédique selon la revendication 1, dans lequel le premier contour d'engagement d'élément œil (28) et le deuxième contour d'engagement d'élément œil (28) comprennent chacun une extension s'étendant latéralement vers l'autre branche de cadre (16, 18), et le premier contour d'engagement d'élément cadre (30) et le deuxième contour d'engagement d'élément cadre (30) comprenant chacun une fente dimensionnée et conformée pour recevoir de manière coulissante une extension de l'élément cadre (12).

5. - Système de plaque et de vis orthopédique selon la revendication 4, dans lequel l'extension du premier contour d'engagement d'élément œil (28) et du deuxième contour d'engagement d'élément œil (28) comprend une forme en section transversale choisie dans le groupe constitué par :
une forme rectangulaire ;
une forme rectangulaire avec des coins arrondis ;
une forme de queue d'aronde ;
une forme partiellement circulaire ;
une forme partiellement elliptique ;
une forme triangulaire ;
une forme trapézoïdale ;
une forme polygonale ; et
une forme avec une ou plusieurs saillies hors plan.

6. - Système de plaque et de vis orthopédique selon la revendication 1, dans lequel l'élément cadre comprend en outre un passage cylindrique statique (76) dimensionné pour recevoir l'introduction tête la première d'une autre tête de vis orthopédique (36) d'une autre vis orthopédique (38) à l'intérieur de celui-ci et pour assurer un ajustement serré avec l'autre tête de vis orthopédique (36) .

7. - Système de plaque et de vis orthopédique selon la revendication 1, comprenant en outre un autre élément œil (14), dans lequel :
l'autre élément œil (14) comprend :
un matériau biocompatible formé pour définir un passage cylindrique (32) à travers l'autre élément œil (14), le passage cylindrique (32) à travers l'autre élément œil (14) étant dimensionné pour recevoir l'introduction tête la première d'une autre tête de vis orthopédique (36) d'une autre vis orthopédique (38) à l'intérieur de celui-ci et pour assurer un ajustement serré avec l'autre tête de vis orthopédique (36) ; et
un troisième contour d'engagement d'élément cadre (30) et un quatrième contour d'engagement d'élément cadre (30) formés sur des côtés opposés de l'autre élément œil (14) ; et
l'élément cadre (12) comprend en outre un autre canal (26) formé par une troisième branche de cadre (22) et une quatrième branche de cadre (24), toutes deux faites d'un matériau biocompatible ;
la troisième branche de cadre (22) comprenant un troisième contour d'engagement d'élément œil (28) formé sur un côté canal de la troisième branche de cadre (22) et apte à engager le troisième contour d'engagement d'élément cadre (30) de l'autre élément œil (14) en engagement coulissant ; et
la quatrième branche de cadre (24) comprenant un quatrième contour d'engagement d'élément œil (28) formé sur un côté canal de la quatrième branche de cadre (24) et apte à engager le quatrième contour d'engagement d'élément cadre (30) de l'autre élément œil (14) en engagement coulissant ;
l'autre élément œil (14) étant contenu à l'intérieur de l'autre canal (26) et apte à coulisser à l'intérieur de celui-ci.

8. - Système de plaque et de vis orthopédique selon la revendication 1, comprenant en outre un autre élément œil (14), dans lequel :
l'autre élément œil (14) comprend :
un matériau biocompatible formé pour définir un passage cylindrique (32) à travers l'autre élément œil (14), le passage cylindrique (32) à travers l'autre élément œil étant dimensionné pour recevoir l'introduction tête la première d'une autre tête de vis orthopédique (36) d'une autre vis orthopédique (38) à l'intérieur de celui-ci et pour assurer un ajustement serré avec l'autre tête de vis orthopédique (36) ; et
un troisième contour d'engagement d'élément cadre (30) et un quatrième contour d'engagement d'élément cadre (30) formés sur des côtés opposés de l'autre élément œil (14) et aptes à engager les premier et deuxième contours d'engagement d'élément œil (28) des première et deuxième jambes de cadre (16, 18) ; et
l'autre élément œil (14) étant contenu à l'intérieur du canal (20) et apte à coulisser à l'intérieur de celui-ci.

9. - Système de plaque et de vis orthopédique selon la revendication 1, dans lequel la vis orthopédique (38) comprend une caractéristique d'entraînement (82) et une caractéristique d'attache de tige (84) aptes à permettre une retenue de la vis orthopédique (38) à l'encontre d'une force dirigée contre la tête de vis orthopédique (36) pendant un ajustement serré de la tête de vis orthopédique (36) dans le passage cylindrique (32).

10. - Système de plaque et de vis orthopédique selon la revendication 1, comprenant en outre une structure d'arrêt de mouvement apte à inhiber au moins de manière sélective un mouvement de coulissement de l'élément œil (14) à l'intérieur du canal (20).

11. - Système de plaque et de vis orthopédique selon la revendication 10, dans lequel la structure d'arrêt de mouvement comprend une structure choisie dans le groupe constitué par :
une structure d'embrayage à rouleau cylindrique ;
une structure d'embrayage à roue libre ;
une structure d'embrayage à billes ;
une structure d'embrayage à rouleau cylindrique avec un ressort d'excitation ;
une structure d'embrayage à roue libre avec un ressort d'excitation ;
une structure d'embrayage à billes avec un ressort d'excitation ;
un mécanisme à cliquet denté ;
un coin auto-activé ;
un élément de came intégré ; et
un ajustement serré entre l'élément œil (14) et le canal (20) causé par un élargissement de l'élément œil (14) à l'introduction de la tête de vis orthopédique (36) dans le passage cylindrique (32).

12. - Système de plaque et de vis orthopédique selon la revendication 1, dans lequel le canal (20) a une forme choisie dans le groupe constitué par une forme à extrémité ouverte et une forme à extrémité fermée.

13. - Système de plaque et de vis orthopédique selon la revendication 1, dans lequel la plaque orthopédique (10) est dimensionnée pour être utilisée dans une procédure de fixation de vertèbre cervicale.

14. - Système de plaque et de vis orthopédique selon la revendication 1, le système comprenant :
la vis orthopédique (38) qui est une première vis orthopédique (38) ayant une première tige filetée (78) s'étendant à partir d'une première tête de vis orthopédique (36) ;
une seconde vis orthopédique (38) ayant une seconde tige filetée (78) s'étendant à partir d'une seconde tête de vis orthopédique (36) ayant une surface sphérique extérieure ;
l'élément œil (14) qui est un premier élément œil (14) ;
un second élément œil (14) comprenant :
un matériau biocompatible formé pour définir un second passage cylindrique (32) à travers le second élément œil (14), le second passage cylindrique (32) à travers le second élément œil (14) étant dimensionné légèrement plus petit que la tête de vis orthopédique (36), ce par quoi le passage cylindrique (32) est apte à recevoir l'introduction tête la première de la seconde tête de vis orthopédique (36) à l'intérieur de celui-ci et à assurer un ajustement serré du second élément œil (14) avec la seconde tête de vis orthopédique (36) ; et
un troisième contour d'engagement d'élément cadre (30) et un quatrième contour d'engagement d'élément cadre (30) formés sur des côtés opposés du second élément œil (14) ; et
le canal (26) de l'élément cadre qui est un premier canal (26), et l'élément cadre comprenant en outre :
un second canal (26) formé par une troisième branche de cadre (22) et une quatrième branche de cadre (24), toutes deux faites d'un matériau biocompatible ;
la troisième branche de cadre (22) comprenant un troisième contour d'engagement d'élément œil (28) formé sur un côté canal de la troisième branche de cadre (22) et apte à engager le troisième contour d'engagement d'élément cadre (30) du second élément œil (14) en engagement coulissant ; et
la quatrième branche de cadre (24) comprenant un quatrième contour d'engagement d'élément oeil (28) formé sur un côté canal de la quatrième branche de cadre (24) et apte à engager le quatrième contour d'engagement d'élément cadre (30) du second élément œil (14) en engagement coulissant ; le second élément œil (14) étant contenu à l'intérieur du second canal (26) et apte à coulisser à l'intérieur de celui-ci.

15. - Système de vis et de plaque orthopédique selon la revendication 14, comprenant en outre un mécanisme de génération de force apte à appliquer une force de compression entre le premier élément oeil (14) et le second élément oeil (14).
